(19) Europäisches Patentamt
European Patent Office
Office européen des brevets

(11) EP 2 225 030 B1

(12) **EUROPÄISCHE PATENTSCHRIFT**

(45) Veröffentlichungstag und Bekanntmachung des Hinweises auf die Patenterteilung:
**18.04.2012 Patentblatt 2012/16**

(21) Anmeldenummer: **08864424.0**

(22) Anmeldetag: **10.12.2008**

(51) Int Cl.:
***B01J 23/835*** (2006.01) ***B01J 23/843*** (2006.01)
***B01J 37/03*** (2006.01) ***B01J 37/18*** (2006.01)
***C07C 209/16*** (2006.01) ***C07C 213/02*** (2006.01)
***C07D 295/023*** (2006.01)

(86) Internationale Anmeldenummer:
**PCT/EP2008/067184**

(87) Internationale Veröffentlichungsnummer:
**WO 2009/080508 (02.07.2009 Gazette 2009/27)**

# (54) VERFAHREN ZUR HERSTELLUNG EINES AMINS

METHOD FOR PRODUCING AN AMINE

PROCÉDÉ DE PRÉPARATION D'UNE AMINE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **21.12.2007 EP 07150407**
**10.10.2008 EP 08166388**

(43) Veröffentlichungstag der Anmeldung:
**08.09.2010 Patentblatt 2010/36**

(73) Patentinhaber: **BASF SE**
**67056 Ludwigshafen (DE)**

(72) Erfinder:
- **KUBANEK, Petr**
  **68163 Mannheim (DE)**
- **MÄGERLEIN, Wolfgang**
  **68165 Mannheim (DE)**
- **SCHWAB, Ekkehard**
  **67434 Neustadt (DE)**
- **MELDER, Johann-Peter**
  **67459 Böhl-Iggelheim (DE)**
- **JULIUS, Manfred**
  **67117 Limburgerhof (DE)**

(56) Entgegenhaltungen:
**EP-A- 0 839 575** **WO-A-03/051508**
**US-A- 4 153 581**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 225 030 B1

## Beschreibung

Die vorliegende Erfindung betrifft zirkoniumdioxid-, kupfer- und nickelhaltige Katalysatoren und ein Verfahren zur Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines zirkoniumdioxid-, kupfer- und nickelhaltigen Katalysators.

Die Verfahrensprodukte finden u.a. Verwendung als Zwischenprodukte bei der Herstellung von Kraftstoffadditiven (US 3,275,554 A; DE 21 25 039 A und DE 36 11 230 A), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

WO 06/069673 A1 (BASF AG) betrifft ein Verfahren zur Direktaminierung von Kohlenwasserstoffen (z.B. Benzol), Katalysatoren die bei der Direktaminierung eingesetzt werden sowie ein Verfahren zur Herstellung dieser Katalysatoren.

In den Katalysatoren sind folgende Metalle oder Metallkombinationen bevorzugt: Ni, Co, Mn, Fe, Ru, Ag und/oder Cu (vgl. Seite 4, Zeilen 10-14).

US 4,153,581 (Habermann) betrifft die Aminierung von Alkoholen, Aldehyden oder Ketonen mittels spezifischer Co/Cu - Katalysatoren, die Fe, Zn und/oder Zr enthalten.

US 4,152,353 (Dow) betrifft die Aminierung von Alkoholen, Aldehyden oder Ketonen mittels spezifischer Ni/Cu - Katalysatoren, die Fe, Zn und/oder Zr enthalten.

Aus der EP 382 049 A1 (BASF AG) sind Katalysatoren, die sauerstoffhaltige Zirkonium-, Kupfer-, Kobalt- und Nickelverbindungen enthalten, und Verfahren zur hydrierenden Aminierung von Alkoholen bekannt. Der bevorzugte Zirkoniumoxidgehalt dieser Katalysatoren beträgt 70 bis 80 Gew.-% (loc. cit.: Seite 2, letzter Absatz; Seite 3, 3. Absatz; Beispiele). Diese Katalysatoren zeichnen sich zwar durch eine gute Aktivität und Selektivität aus, zeigen allerdings verbesserungswürdige Standzeiten.

EP 963 975 A1 und EP 1 106 600 A2 (beide BASF AG) beschreiben Verfahren zur Herstellung von Aminen aus Alkoholen bzw. Aldehyden oder Ketonen und Stickstoffverbindungen unter Verwendung eines Katalysators, dessen katalytisch aktive Masse 22-40 Gew.-% (bzw. 22-45 Gew.-%) sauerstoffhaltige Verbindungen des Zirkoniums, 1-30 Gew.-% sauerstoffhaltige Verbindungen des Kupfers und jeweils 15-50 Gew.-% (bzw. 5-50 Gew.-%) sauerstoffhaltige Verbindungen des Nickels und Kobalts enthält.

Auch WO 031076386 A und EP 1 431 271 A1 (beide BASF AG) lehren Katalysatoren des o.g. Typs für Aminierungen.

WO 03/051508 A1 (Huntsman Petrochemical Corp.) betrifft Verfahren zur Aminierung von Alkoholen unter Verwendung von spezifischen Cu/Ni/Zr/Sn - haltigen Katalysatoren, die in einer weiteren Ausgestaltung Cr statt Zr enthalten (siehe Seite 4, Zeilen 10-16). Die in dieser WO-Anmeldung beschriebenen Katalysatoren enthalten kein Kobalt.

WO 2007/036496 A (BASF AG) beschreibt ein Verfahren zur Herstellung von Aminodiglykol (ADG) und Morpholin durch Umsetzung von Diethylenglykol (DEG) mit Ammoniak in Gegenwart eines Übergangsmetall-Heterogenkatalysators, wobei die katalytisch aktive Masse des Katalysators vor der Behandlung mit Wasserstoff sauerstoffhaltige Verbindungen des Aluminiums und/oder Zirkoniums, Kupfers, Nickels und Kobalts enthält und der Katalysatorformkörper spezifische Dimensionen aufweist.

Fünf Patentanmeldungen mit Anmeldetag 14.07.06 (alle BASF AG), Aktenzeichen EP 06117249.0, 06117251.6, 06117253.2, 06117259.9 und 06117243.3, (WO 2008/006750 A, WO 2008/006748 A, WO 2008/006752 A, WO 2008/006749 A, WO 2008/006754 A) betreffen bestimmte dotierte, zirkoniumdioxid-, kupfer- und nickelhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin.

Die in den Anmeldungen mit den Aktenzeichen 06117249.0, 06117251.6, 06117253.2 beschriebenen Katalysatoren enthalten 10 bis 50 Gew.-%, bevorzugt 16 bis 35 Gew.-% Co.

Sechs parallele europäische Patentanmeldungen mit gleichem Anmeldetag (alle BASF AG) betreffen bestimmte zirkoniumdioxid- und nickelhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin.

Beim Einsatz der sehr aktiven Katalysatoren des Stands der Technik, insbesondere auch der Katalysatoren gemäß EP 963 975 A1 und EP 1 106 600 A2 (s.o.), kann es bei den Edukten (Alkohole, Aldehyde, Ketone) bei erhöhter Temperatur verstärkt zur Decarbonylierung der (gegebenenfalls intermediär entstandenen) Carbonylfunktion kommen. Die Bildung von Methan durch Hydrierung von Kohlenmonoxid (CO) führt aufgrund der großen freiwerdenden Hydrierwärme zu einer 'Durchgehgefahr', d.h. einem unkontrollierten Temperaturanstieg im Reaktor. Wird CO durch Amine abgefangen, kommt es zur Bildung von Methylgruppen-haltigen Nebenkomponenten.

Bei der Aminierung von Diethylenglykol (DEG) kommt es beispielsweise verstärkt zur Bildung von unerwünschtem Methoxyethanol bzw. Methoxyethylamin. Das Methoxyethanol ist giftig, kann aufgrund seiner physikalischen Eigenschaften aus Morpholin nur schlecht abgetrennt werden und kann damit zu Problemen hinsichtlich Spezifikation und Produktqualität führen.

Beim Beispiel der Aminierung von Diethylenglykol (DEG) wird die "Decarbonylierung" insbesondere als die Summe von unerwünschten Komponenten (Methanol, Methoxyethanol, Methoxyethylamin, N-Methylmorpholin und Methoxy-Ethyl-Morpholin) betrachtet, die gemäß dem Reaktionsnetzwerk aus DEG über Methoxyethanol entstehen:

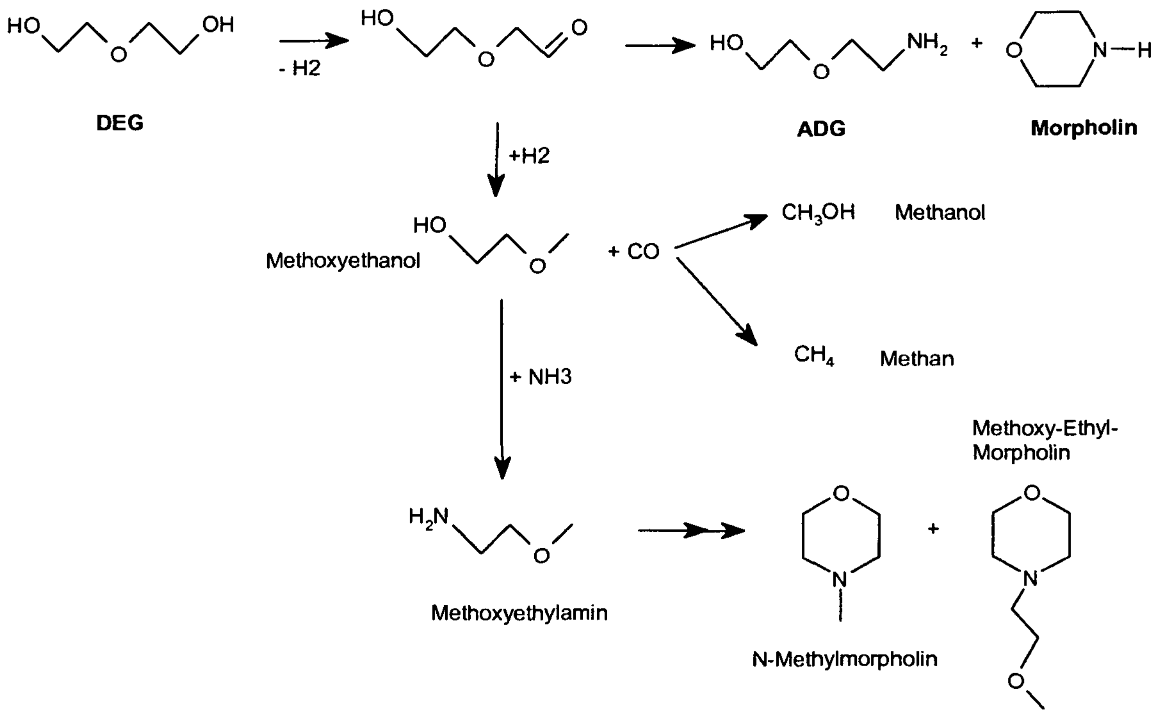


Als Reaktionsmechanismus der Aminierung von primären oder sekundären Alkoholen wird angenommen, dass der Alkohol zunächst an einem Metallzentrum zum entsprechenden Aldehyd dehydriert wird. Hierbei kommt dem Kupfer oder auch Nickel als Dehydrierkomponente vermutlich besondere Bedeutung zu. Werden Aldehyde zur Aminierung eingesetzt, entfällt dieser Schritt.

Der gebildete bzw. eingesetzte Aldehyd kann durch Reaktion mit Ammoniak oder primärem oder sekundärem Amin unter Wasserabspaltung und anschließender Hydrierung aminiert werden. Diese Kondensation des Aldehyds mit der o.g. Stickstoffverbindung wird vermutlich durch saure Zentren des Katalysators katalysiert. In einer unerwünschten Nebenreaktion kann der Aldehyd aber auch decarbonyliert werden, d.h. dass die Aldehydfunktion als CO abgespalten wird. Die Decarbonylierung bzw. Methanisierung findet vermutlich an einem metallischen Zentrum statt. Das CO wird an dem Hydrierkatalysator zu Methan hydriert, so dass die Methanbildung das Ausmaß der Decarbonylierung anzeigt. Durch die Decarbonylierung entstehen die oben erwähnten unerwünschten Nebenprodukte wie z.B. im o.g. Fall Methoxyethanol und/oder Methoxyethylamin.

Es handelt sich bei der erwünschten Kondensation des Aldehyds mit Ammoniak oder primärem oder sekundärem Amin und bei der unerwünschten Decarbonylierung des Aldehyds um Parallelreaktionen, von denen die erwünschte Kondensation vermutlich säurekatalysiert ist, während die unerwünschte Decarbonylierung durch metallische Zentren katalysiert ist.

Der vorliegenden Erfindung lag die Aufgabe zugrunde, die Wirtschaftlichkeit bisheriger Verfahren zur hydrierenden Aminierung von Aldehyden oder Ketonen und der Aminierung von Alkoholen zu verbessern und einem Nachteil oder mehreren Nachteilen des Stands der Technik, insbesondere den o.g. Nachteilen, abzuhelfen. Es sollten Katalysatoren gefunden werden, die technisch in einfacher Weise herzustellen sind und die es erlauben, die o.g. Aminierungen mit hohem Umsatz, hoher Ausbeute, Raum-Zeit-Ausbeuten (RZA), Selektivität, Katalysatorstandzeit bei gleichzeitig hoher mechanischer Stabilität des Katalysatorformkörpers und geringer 'Durchgehgefahr', durchzuführen. Die Katalysatoren sollen demnach eine hohe Aktivität und unter den Reaktionsbedingungen eine hohe chemische und mechanische Stabilität aufweisen. Darüber hinaus sollte der Einsatz der Katalysatoren in entsprechenden Aminierungsverfahren, in denen aufgrund der chemischen Struktur der Edukte lineare und zyklische Verfahrensprodukte resultieren können, mit verbesserter Selektivität zu dem/den linearen Verfahrensproduktlen führen.

[Raum-Zeit-Ausbeuten werden angegeben in ‚Produktmenge / (Katalysatorvolumen • Zeit)' (kg/($l_{Kat.}$ • h)) und/oder ‚Produktmenge / (Reaktorvolumen • Zeit)' (kg/($l_{Reaktor}$ • h)].

Demgemäß wurde ein Verfahren zur Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines zirkoniumdioxid-, kupfer- und nickelhaltigen Katalysators gefunden, welches dadurch gekennzeichnet ist, dass die katalytisch aktive Masse des Katalysators vor dessen Reduktion mit Wasserstoff sauerstoffhaltige Verbindungen des Zirkoniums, Kupfers, Nickels, im Bereich von 0,2 bis 40 Gew.-%, bevorzugt 1 bis 25 Gew.-%, besonders bevorzugt 2 bis 10 Gew.-%, sauerstoffhaltige Verbindungen des Kobalts, berechnet als CoO, im Bereich von 0,1 bis 5 Gew.-%, bevorzugt 0,2 bis 4 Gew.-%, besonders bevorzugt 0,5 bis 3 Gew.-%, sauerstoffhaltige Verbindungen des Eisens, berechnet als $Fe_2O_3$, und im Bereich von 0,1 bis 5 Gew.-%, bevorzugt 0,3 bis 4,5 Gew.-%, besonders bevorzugt 0,5 bis 4 Gew.-%, sauerstoffhaltige Verbindungen des Bleis, Zinns, Bismuths und/oder Antimons, jeweils berechnet als PbO, SnO, $Bi_2O_3$ bzw. $Sb_2O_3$, enthält.

Darüber hinaus wurden Katalysatoren gefunden, deren katalytisch aktive Masse vor deren Reduktion mit Wasserstoff sauerstoffhaltige Verbindungen des Zirkoniums, Kupfers, Nickels, im Bereich von 0,2 bis 40 Gew.-%, bevorzugt 1 bis 25 Gew.-%, besonders bevorzugt 2 bis 10 Gew.-%, sauerstoffhaltige Verbindungen des Kobalts, berechnet als CoO, im Bereich von 0,1 bis 5 Gew.-%, bevorzugt 0,2 bis 4 Gew.-%, besonders bevorzugt 0,5 bis 3 Gew.-%, sauerstoffhaltige Verbindungen des Eisens, berechnet als $Fe_2O_3$, und im Bereich von 0,1 bis 5 Gew.-%, bevorzugt 0,3 bis 4,5 Gew.-%, besonders bevorzugt 0,5 bis 4 Gew.-%, sauerstoffhaltige Verbindungen des Bleis, Zinns, Bismuths und/oder Antimons, jeweils berechnet als PbO, SnO, $Bi_2O_3$ bzw. $Sb_2O_3$, enthält.

Insbesondere wurden Katalysatoren, deren katalytisch aktive Masse vor deren Reduktion mit Wasserstoff im Bereich von 20 bis 85 Gew.-% sauerstoffhaltige Verbindungen des Zirkoniums, berechnet als $ZrO_2$, 0,2 bis 25 Gew.-% sauerstoffhaltige Verbindungen des Kupfers, berechnet als CuO, 0,2 bis 45 Gew.-% sauerstoffhaltige Verbindungen des Nickels, berechnet als NiO, und 0,2 bis 40 Gew.-%, bevorzugt 1 bis 25 Gew.-%, besonders bevorzugt 2 bis 10 Gew.-%, sauerstoffhaltige Verbindungen des Kobalts, berechnet als CoO,
0,1 bis 5 Gew.-%, bevorzugt 0,2 bis 4 Gew.-%, besonders bevorzugt 0,5 bis 3 Gew.-%, sauerstoffhaltige Verbindungen des Eisens, berechnet als $Fe_2O_3$, und 0,1 bis 5,0 Gew.-%, bevorzugt 0,3 bis 4,5 Gew.-%, besonders 0,5 bis 4 Gew.-%, sauerstoffhaltige Verbindungen des Bleis, Zinns, Bismuths und/oder Antimons, jeweils berechnet als PbO, SnO, $Bi_2O_3$ bzw. $Sb_2O_3$, enthält,
und ihre Verwendung im o.g. Aminierungsverfahren, insbesondere im Verfahren zur Umsetzung von DEG mit Ammoniak, gefunden.

Unter den o.g. Dotierungskomponenten Pb, Sn, Bi und Sb ist Pb (Blei) besonders bevorzugt.

In einer ganz besonders bevorzugten Ausführungsform enthält die katalytisch aktive Masse des erfindungsgemäßen und im erfindungsgemäßen Verfahren eingesetzten Katalysators, vor deren Reduktion mit Wasserstoff, im Bereich von 2 bis 10 Gew.-% sauerstoffhaltige Verbindungen des Kobalts, berechnet als CoO, und 0,1 bis 5,0 Gew.-%, bevorzugt 0,3 bis 4,5 Gew.-%, besonders 0,5 bis 4 Gew.-%, sauerstoffhaltige Verbindungen des Bleis, berechnet als PbO.

Alle Angaben zur Zusammensetzung der katalytisch aktiven Masse der erfindungsgemäßen und im erfindungsgemäßen Verfahren verwendeten Katalysatoren beziehen sich auf die katalytisch aktive Masse vor deren Reduktion mit Wasserstoff.

Erfindungsgemäß wurde erkannt, dass die Aktivität des Katalysators zur Aminierung von primären oder sekundären Alkoholen, Aldehyden und/oder Ketonen in Gegenwart von $H_2$, z.B. die Aminierung von Diethylenglykol (DEG) mit Ammoniak zu Aminodiglykol und Morpholin, durch den spezifischen Gehalt der Zirkonium-Kupfer-NickelKatalysatoren an Kobalt und Eisen und den zusätzlichen Gehalt an Blei, Zinn, Bismuth und/oder Antimon, im wesentlichen zumindest gleich bleibt, gleichzeitig aber das Ausmaß der unerwünschten Decarbonylierungsreaktion abnimmt und damit die Selektivität der Aminierungsreaktion zunimmt.

Das Verfahren kann kontinuierlich oder diskontinierlich durchgeführt werden. Bevorzugt ist eine kontinuierliche Fahrweise.

Für die Synthese in der Gasphase werden die Edukte gezielt, bevorzugt in einem Kreisgasstrom, verdampft und gasförmig dem Reaktor zugeführt. Geeignete Amine für eine Gasphasensynthese sind Amine, die aufgrund ihrer Siedepunkte und der Siedepunkte ihrer Edukte verfahrenstechnisch im Rahmen der Prozessparameter in der Gasphase gehalten werden können. Das Kreisgas dient zum einen der Verdampfung der Edukte und zum anderen als Reaktionspartner für die Aminierung.

In der Kreisgasfahrweise werden die Ausgangsstoffe (Alkohol, Aldehyd und/oder Keton, Wasserstoff und die Stickstoffverbindung) in einem Kreisgasstrom verdampft und gasförmig dem Reaktor zugeführt.

Die Edukte (Alkohol, Aldehyd und/oder Keton, die Stickstoffverbindung) können auch als wässrige Lösungen verdampft und mit dem Kreisgasstrom auf das Katalysatorbett geleitet werden.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Alternativ erfolgt die Umsetzung vorteilhaft in einem Rohrbündelreaktor oder in einer Monostranganlage.

Bei einer Monostranganlage kann der Rohrreaktor, in dem die Umsetzung erfolgt, aus einer Hintereinanderschaltung mehrerer (z.B. zweier oder dreier) einzelner Rohrreaktoren bestehen. Optional ist hier vorteilhaft eine Zwischeneinspeisung von Feed (enthaltend das Edukt und/oder Ammoniak und/oder $H_2$) und/oder Kreisgas und/oder Reaktoraustrag aus einem nachgeschalteten Reaktor möglich.

Die Kreisgasmenge liegt bevorzugt im Bereich von 40 bis 1500 $m^3$ (bei Betriebsdruck) / [$m^3$ Katalysator (Schüttvolumen) • h], insbesondere im Bereich von 100 bis 700 $m^3$ (bei Betriebsdruck) / [$m^3$ Katalysator (Schüttvolumen) • h].

Das Kreisgas enthält bevorzugt mindestens 10, besonders 50 bis 100, ganz besonders 80 bis 100, Vol.% $H_2$.

Für die Synthese in der Flüssigphase sind alle Edukte und Produkte geeignet, welche schwer verdampfbar oder thermisch labil sind. In diesen Fällen kommt als weiterer Vorteil hinzu, dass das auf eine Verdampfung und Rekondensation des Amins im Prozess verzichtet werden kann.

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.

In diesem Zusammenhang wird das oxidische Trägermaterial Zirkoniumdioxid ($ZrO_2$) als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper — beispielsweise als Tabletten, Kugeln, Ringe, Extrudate (z. B. Stränge) — im Reaktor anordnet.

Die Konzentrationsangaben (in Gew.-%) der Komponenten des Katalysators beziehen sich jeweils — falls nicht anders angegeben — auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o. g. Katalysatorträgermaterialien definiert und enthält im wesentlichen die folgenden Bestandteile:

Zirkoniumdioxid ($ZrO_2$), sauerstoffhaltige Verbindungen des Kupfers, Nickels und Kobalts und Eisens und sauerstoffhaltige Verbindungen des Bleis, Zinns, Bismuths und/oder Antimons.

Die Summe der o. g. Bestandteile der katalytisch aktiven Masse beträgt üblicherweise 70 bis 100 Gew.-%, bevorzugt 80 bis 100 Gew.-%, besonders bevorzugt 90 bis 100 Gew.-%, besonders > 95 Gew.-%, ganz besonders > 98 Gew.-%, insbesondere > 99 Gew.-%, z.B. besonders bevorzugt 100 Gew.-%.

Die katalytisch aktive Masse der erfindungsgemäßen und im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten. Beispiele für solche Elemente bzw. deren Verbindungen sind:

Übergangsmetalle, wie Mn bzw. $MnO_2$, W bzw. Wolframoxide, Ta bzw. Tantaloxide, Nb bzw. Nioboxide oder Nioboxalat, V bzw. Vanadiumoxide bzw. Vanadylpyrophosphat;
Lanthanide, wie Ce bzw. $CeO_2$ oder Pr bzw. $Pr_2O_3$; Alkalimetalloxide, wie $Na_2O$; Alkalimetallcarbonate, wie $Na_2CO_3$; Erdalkalimetalloxide, wie SrO; Erdalkalimetallcarbonate, wie $MgCO_3$, $CaCO_3$ und $BaCO_3$; Boroxid ($B_2O_3$).

Bevorzugt enthält die katalytisch aktive Masse der erfindungsgemäßen und im erfindungsgemäßen Verfahren eingesetzten Katalysatoren kein Rhenium, kein Ruthenium und/oder kein Zink, jeweils weder in metallischer (Oxidationsstufe 0) noch in einer ionischen, insb. oxidierten, Form.

Bevorzugt enthält die katalytisch aktive Masse der erfindungsgemäßen und im erfindungsgemäßen Verfahren eingesetzten Katalysatoren kein Silber und/oder Molybdän, jeweils weder in metallischer (Oxidationsstufe 0) noch in einer ionischen, insb. oxidierten, Form.

In einer besonders bevorzugten Ausführungsform enthält die katalytisch aktive Masse der erfindungsgemäßen und im erfindungsgemäßen Verfahren eingesetzten Katalysatoren keine weitere katalytisch aktive Komponente, weder in elementarer noch in ionischer Form.

In der besonders bevorzugten Ausführungsform ist die katalytisch aktive Masse nicht mit weiteren Metallen oder Metallverbindungen dotiert.

Bevorzugt sind jedoch aus der Metallgewinnung von Cu, Co, Ni, Pb, Sn, Bi, Sb herrührende übliche Begleit-Spurenelemente hiervon ausgenommen.

Die katalytisch aktive Masse des Katalysators enthält vor dessen Reduktion mit Wasserstoff im Bereich von 0,2 bis 40 Gew.-%, besonders im Bereich von 1 bis 25 Gew.-%, weiter besonders im Bereich von 2 bis 10 Gew.-%, sauerstoffhaltige Verbindungen des Kobalts, berechnet als CoO.

Die katalytisch aktive Masse des Katalysators enthält vor dessen Reduktion mit Wasserstoff im Bereich von 0,1 bis 5 Gew.-%, besonders im Bereich von 0,2 bis 4 Gew.-%, weiter besonders im Bereich von 0,5 bis 3 Gew.-%, sauerstoffhaltige Verbindungen des Eisens, berechnet als $Fe_2O_3$.

Die katalytisch aktive Masse des Katalysators enthält vor dessen Reduktion mit Wasserstoff weiterhin bevorzugt im Bereich von 20 bis 85 Gew.-%, besonders 25 bis 70 Gew.-%, weiter besonders 30 bis 60 Gew.-%, sauerstoffhaltige Verbindungen des Zirkoniums, berechnet als $ZrO_2$,

0,2 bis 25 Gew.-%, besonders 3 bis 20 Gew.-%, weiter besonders 5 bis 15 Gew.-%, sauerstoffhaltige Verbindungen des Kupfers, berechnet als CuO,

0,2 bis 45 Gew.-%, besonders 10 bis 40 Gew.-%, weiter besonders 25 bis 35 Gew.-%, sauerstoffhaltige Verbindungen des Nickels, berechnet als NiO, und

0,1 bis 5,0 Gew.-%, besonders 0,3 bis 4,5 Gew.-%, weiter besonders 0,5 bis 4 Gew.-%, sauerstoffhaltige Verbindungen des Bleis, Zinns, Bismuths und/oder Antimons, jeweils berechnet als PbO, SnO, $Bi_2O_3$ bzw. $Sb_2O_3$.

Das Molverhältnis von Nickel zu Kupfer beträgt bevorzugt größer 1, besonders bevorzugt größer 1,2, weiter besonders bevorzugt im Bereich von 1,8 bis 8,5.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Komponenten mit Wasser und nachfolgendes Extrudieren und Tempern (Wärmebehandlung) der so erhaltenen Masse erhältlich.

Bevorzugt werden zur Herstellung der erfindungsgemäßen Katalysatoren Fällungsmethoden angewandt. So können sie beispielsweise durch eine gemeinsame Fällung der Nickel-, Kobalt- und Kupfer- und Eisen- und Dotierungs-Komponenten aus einer diese Elemente enthaltenden, wässrigen Salzlösung mittels Basen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und - silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Zirkoniumverbindungen aus wässrigen Zirkoniumsalzlösungen mittels Basen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Base, - beispielsweise Natriumcarbonat, Natriumhydroxid, Kaliumcarbonat oder Kaliumhydroxid - versetzt, bis die Fällung vollständig ist. Es kann auch mit Alkalimetall-freien Basen wie Ammoniak, Ammoniumcarbonat, Ammoniumhydrogencarbonat, Ammoniumcarbamat, Ammoniumoxalat, Ammoniummalonat, Urotropin, Harnstoff, etc. gearbeitet werden. Die Art der verwendeten Salze ist im allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden zu den erfindungsgemäßen Katalysatoren wie üblich weiterverarbeitet. Zunächst werden die Niederschläge gewaschen. Über die Dauer des Waschvorgangs und über die Temperatur und Menge des Waschwassers kann der Gehalt an Alkalimetall, das durch die als Fällungsmittel eventuell verwendete (Mineral)base zugeführt wurde, beeinflusst werden. Im Allgemeinen wird durch Verlängerung der Waschzeit oder Erhöhung der Temperatur des Waschwassers der Gehalt an Alkalimetall abnehmen. Nach dem Waschen wird das Fällgut im allgemeinen bei 80 bis 200 °C, vorzugsweise bei 100 bis 150 °C, getrocknet und danach calciniert. Die Calcinierung wird im allgemeinen bei Temperaturen zwischen 300 und 800 °C, vorzugsweise bei 400 bis 600 °C, insbesondere bei 450 bis 550 °C ausgeführt.

Die erfindungsgemäßen Katalysatoren können auch durch Tränkung von Zirkoniumdioxid ($ZrO_2$) das beispielsweise in Form von Pulver oder Formkörpern, wie Strängen, Tabletten, Kugeln oder Ringen, vorliegt, hergestellt werden.

Das Zirkoniumdioxid wird beispielsweise in der amorphen, monoklinen oder tetragonalen Form, bevorzugt in der monoklinen Form eingesetzt.

Die Herstellung von Formkörpern kann nach den üblichen Verfahren erfolgen.

Die Tränkung erfolgt ebenfalls nach den üblichen Verfahren, wie z. B. in A. B. Stiles, Catalyst Manufacture - Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und optional kalziniert.

Die Tränkung kann nach der sogenannten "incipient wetness"-Methode erfolgen, bei der das Zirkoniumdioxid entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und optional zu kalzinieren. Die mehrstufige Tränkung ist vorteilhaft besonders dann anzuwenden, wenn das Zirkoniumdioxid mit einer größeren Metallmenge beaufschlagt werden soll.

Zur Aufbringung der Metallkomponenten auf das Zirkoniumdioxid kann die Tränkung gleichzeitig mit allen Metallsalzen oder in beliebiger Reihenfolge der einzelnen Metallsalze nacheinander erfolgen.

Anschließend werden die durch Tränkung hergestellten Katalysatoren getrocknet und bevorzugt auch calciniert, z.B. bei den bereits oben angegebenen Calciniertemperaturbereichen.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Presse zu Formlingen, z. B. Tabletten, verpresst und tempert. Die Tempertemperaturen entsprechen dabei bevorzugt den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die z.B. wie oben beschreiben hergestellten Katalysatoren werden als solche gelagert und ggf. gehandelt. Vor ihrem Einsatz als Katalysatoren werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden.

Zur Vorreduktion werden die Katalysatoren zunächst bei bevorzugt 150 bis 200 °C über einen Zeitraum von z.B. 12 bis 20 Stunden einer Stickstoff-WasserstoffAtmosphäre ausgesetzt und anschließend noch bis zu ca. 24 Stunden bei bevorzugt 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Ein weiterer Vorteil der erfindungsgemäßen Katalysatoren ist deren mechanische Stabilität, d.h. deren Härte. Die mechanische Stabilität kann durch die Messung der sogenannten Seitendruckfestigkeit bestimmt werden. Hierzu wird der Katalysatorformkörper, z. B. die Katalysatortablette, zwischen zwei parallelen Platten mit zunehmender Kraft belastet, wobei diese Belastung z. B. auf der Mantelseite von Katalysatortabletten erfolgen kann, bis ein Bruch des Katalysatorformkörpers eintritt. Die beim Bruch des Katalysatorformkörpers registrierte Kraft ist die Seitendruckfestigkeit.

Das erfindungsgemäße Verfahren wird bevorzugt kontinuierlich durchgeführt, wobei der Katalysator bevorzugt als Festbett im Reaktor angeordnet ist. Dabei ist sowohl eine Anströmung des Katalysatorfestbetts von oben als auch von unten möglich. Der Gasstrom wird dabei durch Temperatur, Druck und Menge so eingestellt, dass auch schwerer siedende (hoch siedende) Reaktionsprodukte in der Gasphase verbleiben.

Das Aminierungsmittel kann bezüglich der zu aminierenden alkoholischen Hydroxylgruppe bzw. Aldehydgruppe bzw. Ketogruppe in stöchiometrischen, unter- oder überstöchiometrischen Mengen eingesetzt werden.

Bevorzugt wird im Falle der Aminierung von Alkoholen, Aldehyden oder Ketonen mit primären oder sekundären Aminen das Amin in ca. stöchiometrischer Menge oder geringfügig überstöchiometrischer Menge pro Mol zu aminierender alkoholischer Hydroxylgruppe, Aldehydgruppe oder Ketogruppe eingesetzt.

Die Aminkomponente (Stickstoffverbindung) wird bevorzugt in der 0,90- bis 100-fachen molaren Menge, insbesondere in der 1,0- bis 10-fachen molaren Menge, jeweils bezogen auf den/das eingesetzte/n Alkohol, Aldehyd und/oder Keton eingesetzt.

Speziell Ammoniak wird im allgemeinen mit einem 1,5 bis 250-fachen, bevorzugt 2 bis 100-fachen, insbesondere 2 bis 10-fachen molaren Überschuss pro Mol umzusetzender alkoholischer Hydroxylgruppe, Aldehydgruppe oder Ketogruppe eingesetzt. Höhere Überschüsse sowohl an Ammoniak als auch an primären oder sekundären Aminen sind möglich.

Bevorzugt wird eine Abgasmenge von 5 bis 800 Normkubikmeter/h, insbesondere 20 bis 300 Normkubikmeter/h, gefahren.

Die Aminierung der primären oder sekundären Alkoholgruppen, Aldehydgruppen oder Ketogruppen des Edukts

kann in der Flüssigphase oder in der Gasphase durchgeführt werden. Bevorzugt ist das Festbettverfahren in der Gasphase.

Beim Arbeiten in der Flüssigphase leitet man die Edukte (Alkohol, Aldehyd oder Keton plus Ammoniak oder Amin) simultan in flüssiger Phase bei Drücken von im allgemeinen 5 bis 30 MPa (50-300 bar), bevorzugt 5 bis 25 MPa, besonders bevorzugt 15 bis 25 MPa, und Temperaturen von im allgemeinen 80 bis 350 °C, besonders 100 bis 300 °C, bevorzugt 120 bis 270 °C, besonders bevorzugt 130 bis 250 °C, insbesondere 170 bis 230 °C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im allgemeinen im Bereich von 0,05 bis 5, bevorzugt 0,1 bis 2, besonders bevorzugt 0,2 bis 0,6, kg Alkohol, Aldehyd oder Keton pro Liter Katalysator (Schüttvolumen) und Stunde. Gegebenenfalls kann eine Verdünnung der Edukte mit einem geeigneten Lösungsmittel, wie Tetrahydrofuran, Dioxan, N-Methylpyrrolidon oder Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Beim Arbeiten in der Gasphase werden die gasförmigen Edukte (Alkohol, Aldehyd oder Keton plus Ammoniak oder Amin) in einem zur Verdampfung ausreichend groß gewählten Gasstrom, bevorzugt Wasserstoff, bei Drücken von im allgemeinen 0,1 bis 40 MPa (1 bis 400 bar), bevorzugt 0,1 bis 10 MPa, besonders bevorzugt 0,1 bis 5 MPa, in Gegenwart von Wasserstoff über den Katalysator geleitet. Die Temperaturen für die Aminierung von Alkoholen betragen im allgemeinen 80 bis 350 °C, besonders 100 bis 300 °C, bevorzugt 120 bis 270 °C, besonders bevorzugt 160 bis 250 °C. Die Reaktionstemperaturen bei der hydrierenden Aminierung von Aldehyden und Ketonen betragen im allgemeinen 80 bis 350 °C, besonders 90 bis 300 °C, bevorzugt 100 bis 250 °C. Es ist dabei sowohl eine Anströmung des Katalysatorfestbetts von oben als auch von unten möglich. Den erforderlichen Gasstrom erhält man bevorzugt durch eine Kreisgasfahrweise.

Die Katalysatorbelastung liegt im allgemeinen im Bereich von 0,01 bis 2, bevorzugt 0,05 bis 0,5, kg Alkohol, Aldehyd oder Keton pro Liter Katalysator (Schüttvolumen) und Stunde.

Der Wasserstoff wird der Reaktion im allgemeinen in einer Menge von 5 bis 400 l, bevorzugt in einer Menge von 50 bis 200 l pro Mol Alkohol-, Aldehyd- oder Ketonkomponente zugeführt, wobei die Literangaben jeweils auf Normalbedingungen umgerechnet wurden (S.T.P.).

Die Aminierung von Aldehyden bzw. Ketonen unterscheidet sich in der Durchführung von der Aminierung von Alkoholen dadurch, dass bei der Aminierung von Aldehyden und Ketonen mindestens stöchiometrische Mengen an Wasserstoff vorhanden sein müssen.

Sowohl beim Arbeiten in der Flüssigphase als auch beim Arbeiten in der Gasphase ist die Anwendung höherer Temperaturen und höherer Gesamtdrücke und Katalysatorbelastungen möglich. Der Druck im Reaktionsgefäß, welcher sich aus der Summe der Partialdrücke des Aminierungsmittels, des Alkohols, Aldehyds bzw. Ketons und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe, Aldehydgruppe bzw. Ketogruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z.B. durch eine fraktionierende Rektifikation. Geeignete Aufarbeitungsverfahren sind z.B. in EP 1 312 600 A und EP 1 312 599 A (beide BASF AG) beschrieben. Das überschüssige Aminierungsmittel und der Wasserstoff werden vorteilhaft wieder in die Reaktionszone zurückgeführt. Das gleiche gilt für die eventuell nicht vollständig umgesetzte Alkohol-, Aldehyd- bzw. Ketonkomponente.

Unumgesetzte Edukte und gegebenenfalls anfallende geeignete Nebenprodukte können wieder in die Synthese zurückgeführt werden. Nicht umgesetzte Edukte können in diskontinuierlicher oder kontinuierlicher Fahrweise nach Kondensation der Produkte im Abscheider in dem Kreisgasstrom erneut über das Katalysatorbett geströmt werden.

Aminierungsmittel im erfindungsgemäßen Verfahren sind neben Ammoniak primäre und sekundäre Amine.

Mit dem erfindungsgemäßen Verfahren herstellbar sind z.B. Amine der Formel I

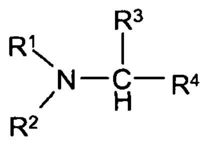

(I),

in der

| | |
|---|---|
| $R^1$, $R^2$ | Wasserstoff (H), Alkyl, wie $C_{1-20}$-Alkyl, Cycloalkyl, wie $C_{3-12}$-Cycloalkyl, Alkoxyalkyl, wie $C_{2-30}$-Alkoxyalkyl, Dialky- laminoalkyl, wie $C_{3-30}$-Dialkylaminoalkyl, Aryl, Aralkyl, wie $C_{7-20}$-Aralkyl, und Alkylaryl, wie $C_{7-20}$-Alkylaryl, oder gemeinsam $-(CH_2)_j-X-(CH_2)_k-$, |
| $R^3$, $R^4$ | Wasserstoff (H), Alkyl, wie $C_{1-20}$-Alkyl, Cycloalkyl, wie $C_{3-12}$-Cycloalkyl, Hydroxyalkyl, wie $C_{1-20}$-Hydroxyalkyl, Aminoalkyl, wie $C_{1-20}$-Aminoalkyl, Hydroxyalkylaminoalkyl, wie $C_{2-20}$- Hydroxyalkylaminoalkyl, Alkoxyalkyl, wie $C_{2-30}$-Alkoxyalkyl, Dialkylaminoalkyl, wie $C_{3-30}$-Dialkylaminoalkyl, Alkylaminoalkyl, wie $C_{2-30}$-Alkylaminoalkyl, $R^5-(OCR^6R^7CR^8R^9)_n-(OCR^6R^7)$, Aryl, Heteroaryl, Aralkyl, wie $C_{7-20}$-Aralkyl, Heteroarylalkyl, wie $C_{4-20}$-Heteroarylalkyl, Alkylaryl, wie $C_{7-20}$-Alkylaryl, Alkylheteroaryl, wie $C_{4-20}$-Alkylheteroaryl, und $Y-(CH_2)_m-NR^5-(CH_2)_q$ oder gemeinsam $-(CH_2)_l-X-(CH_2)_m-$ oder |
| $R^2$ und $R^4$ | gemeinsam $-(CH_2)_l-X-(CH_2)_m-$, |
| $R^5$, $R^{10}$ | Wasserstoff (H), Alkyl, wie $C_{1-4}$-Alkyl, Alkylphenyl, wie $C_{7-40}$-Alkylphenyl, |
| $R^6$, $R^7$, $R^8$, $R^9$ | Wasserstoff (H), Methyl oder Ethyl, |
| X | $CH_2$, $CHR^5$, Sauerstoff (O), Schwefel (S) oder $NR^5$, |
| Y | $N(R^{10})_2$, Hydroxy, $C_{2-20}$-Alkylaminoalkyl oder $C_{3-20}$-Dialkylaminoalkyl, |
| n | eine ganze Zahl von 1 bis 30 und |
| j, k, l, m, q | eine ganze Zahl von 1 bis 4, |

bedeuten.

Das erfindungsgemäße Verfahren findet daher bevorzugt zur Herstellung eines Amins I Anwendung, indem man einen primären oder sekundären Alkohol der Formel II

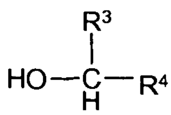

(II)

und/oder Aldehyd und/oder Keton der Formel VI bzw. VII

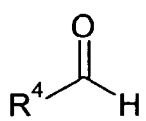

(VI)

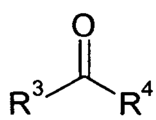

(VII)

mit einer Stickstoffverbindung der Formel III

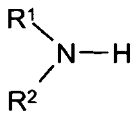

(III),

wobei $R^1$, $R^2$, $R^3$ und $R^4$ die oben genannten Bedeutungen haben, umsetzt.

Bei dem Eduktalkohol kann es sich auch um einen Aminoalkohol handeln, z.B. einem Aminoalkohol gemäß der Formel II.

Wie aus den Definitionen für die Reste $R^2$ und $R^4$ hervorgeht, kann die Umsetzung auch intramolekular in einem entsprechenden Aminoalkohol, Aminoketon oder Aminoaldehyd erfolgen.

Zur Herstellung des Amins I wird demnach rein formal ein Wasserstoffatom der Stickstoffverbindung III durch den Rest $R^4(R^3)CH$- unter Freisetzung von einem Moläquivalent Wasser ersetzt.

Das erfindungsgemäße Verfahren findet auch bevorzugt Anwendung bei der Herstellung eines zyklischen Amins der Formel IV

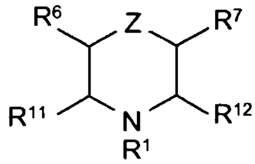

(IV),

in der

| | |
|---|---|
| $R^{11}$ und $R^{12}$ | Wasserstoff (H), Alkyl, wie $C_1$- bis $C_{20}$-Alkyl, Cycloalkyl, wie $C_3$- bis $C_{12}$-Cycloalkyl, Aryl, Heteroaryl, Aralkyl, wie $C_7$- bis $C_{20}$-Aralkyl, und Alkylaryl, wie $C_7$- bis $C_{20}$-Alkylaryl, |
| Z | $CH_2$, $CHR^5$, Sauerstoff (O), $NR^5$ oder $NCH_2CH_2OH$ bedeuten und |
| $R^1$, $R^6$, $R^7$ | die oben genannten Bedeutungen haben, |

durch Umsetzung eines Alkohols der Formel V

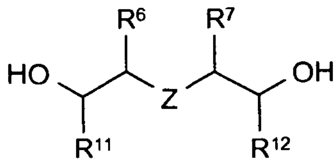

(V)

mit Ammoniak oder einem primären Amin der Formel VIII

$R^1$- $NH_2$ (VIII).

Die Substituenten $R^1$ bis $R^{12}$, die Variablen X, Y, Z und die Indizes j, k, l, m, n und q in den Verbindungen I, II, III, IV, V, VI und VII haben unabhängig voneinander folgende Bedeutungen:

$R^1$, $R^2$, $R^3$, $R^4$, $R^5$, $R^6$, $R^7$, $R^8$, $R^9$, $R^{10}$, $R^{11}$, $R^{12}$:

- Wasserstoff (H),

$R^3$, $R^4$:

- Alkyl, wie $C_{1-20}$-Alkyl, bevorzugt $C_{1-14}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl,

- Hydroxyalkyl, wie $C_{1-20}$-Hydroxyalkyl, bevorzugt $C_{1-8}$-Hydroxyalkyl, besonders bevorzugt $C_{1-4}$-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-(Hydroxymethyl)ethyl,

- Aminoalkyl, wie $C_{1-20}$-Aminoalkyl, bevorzugt $C_{1-8}$-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,

- Hydroxyalkylaminoalkyl, wie $C_{2-20}$-Hydroxyalkylaminoalkyl, bevorzugt $C_{3-8}$-Hydroxyalkylaminoalkyl, wie (2-Hydroxyethylamino)methyl, 2-(2-Hydroxyethylamino)ethyl und 3-(2-Hydroxyethylamino)propyl,

- $R^5$-$(OCR^6R^7CR^8R^9)_n$-$(OCR^6R^7)$, bevorzugt $R^5$-$(OCHR^7CHR^9)_n$-$(OCR^6R^7)$, besonders bevorzugt $R^5$-$(OCH_2$-$CHR^9)_n$-$(OCR^6R^7)$,

- Alkylaminoalkyl, wie $C_{2-30}$-Alkylaminoalkyl, bevorzugt $C_{2-20}$-Alkylaminoalkyl, besonders bevorzugt $C_{2-8}$-Alkylaminoalkyl, wie Methylaminomethyl, 2-Methylaminoethyl, Ethylaminomethyl, 2-Ethylaminoethyl und 2-(iso-Propylamino)ethyl, $(R^5)HN$-$(CH_2)_q$,

- Y-$(CH_2)_m$-$NR^5$-$(CH_2)_q$,

- Heteroarylalkyl, wie $C_{4-20}$-Heteroarylalkyl, wie Pyrid-2-yl-methyl, Furan-2-yl-methyl, Pyrrol-3-yl-methyl und Imidazol-2-yl-methyl,

- Alkylheteroaryl, wie $C_{4-20}$-Alkylheteroaryl, wie 2-Methyl-3-pyridinyl, 4,5-Dimethyl-imidazol-2-yl, 3-Methyl-2-furanyl und 5-Methyl-2-pyrazinyl,

- Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,

$R^1$, $R^2$, R3, $R_{4:}$

- Cycloalkyl, wie $C_{3-12}$-Cycloalkyl, bevorzugt $C_{3-8}$-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,

- Alkoxyalkyl, wie $C_{2-30}$-Alkoxyalkyl, bevorzugt $C_{2-20}$-Alkoxyalkyl, besonders bevorzugt $C_{2-8}$-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt $C_{2-4}$-Alkoxyalkyl,

- Dialkylaminoalkyl, wie $C_{3-30}$-Dialkylaminoalkyl, bevorzugt $C_{3-20}$-Dialkylaminoalkyl, besonders bevorzugt $C_{3-10}$-Dialkylaminoalkyl, wie N,N-Dimethylaminomethyl, (N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Di-iso-propylamino)ethyl, 3-(N,N-Dimethylamino)propyl, $(R^5)_2N$-$(CH_2)_q$,

- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,

- Alkylaryl, wie $C_{7-20}$-Alkylaryl, bevorzugt $C_{7-12}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,

- Aralkyl, wie $C_{7-20}$-Aralkyl, bevorzugt $C_{7-12}$-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,

- $R^3$ und $R^4$ oder $R^2$ und $R^4$ gemeinsam eine -$(CH_2)_l$-X-$(CH_2)_m$- Gruppe, wie - $(CO_2)_3$-, -$(CH_2)_4$-, -$(CH_2)_5$-, -$(CH_2)_6$-, -$(CH_2)_7$-, -$(CH_2)$-O-$(CH_2)_2$-, -$(CH_2)$-$NR^5$-$(CH_2)_2$-, -$(CH_2)$-$CHR^5$-$(CH_2)_2$-, -$(CH_2)_2$-O-$(CH_2)_2$-, -$(CH_2)_2$-$NR^5$-$(CH_2)_2$-, -$(CH_2)_2$-$CHR^5$-$(CH_2)_2$-, -$CH_2$-O-$(CH_2)_3$-, -$CH_2$-$NR^5$-$(CH_2)_3$-, -$CH_2$-$CHR^5$-$(CH_2)_3$-,

$R^1$, $R^2$:

- Alkyl, wie $C_{1-20}$-Alkyl, bevorzugt $C_{1-8}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, besonders bevorzugt $C_{1-4}$-Alkyl, oder

- $R^1$ und $R^2$ gemeinsam eine $-(CH_2)_jX-(CH_2)_k-$ Gruppe, wie $-(CH_2)_3-$, $-(CH_2)_4-$, - $(CH_2)_5-$, $-(CH_2)_6-$, $-(CH_2)_7-$, $-(CH_2)-O-(CH_2)_2-$, $-(CH_2)-NR^5-(CH_2)_2-$, $-(CH_2)-CHR^5-(CH_2)_2-$, $-(CH_2)_2-O-(CH_2)_2-$, $-(CH_2)_2-NR^5-(CH_2)_2-$, $-(CH_2)_2-CHR^5-(CH_2)_2-$, $-CH_2-O-(CH_2)_3-$, $-CH_2-NR^5-(CH_2)_3-$, $-CH_2-CHR^5-(CH_2)_3-$,

$R^5$, $R^{10}$:

- Alkyl, bevorzugt $C_{1-4}$-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,

- Alkylphenyl, bevorzugt $C_{7-40}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl, insbesondere $C_{7-20}$-Alkylphenyl,

$R^6$, $R^7$, $R^8$, $R^9$:

- Methyl oder Ethyl, bevorzugt Methyl,

$R^{11}$, $R^{12}$:

- Alkyl, wie $C_1$- bis $C_{20}$-Alkyl, Cycloalkyl, wie $C_3$- bis $C_{12}$-Cycloalkyl, Aryl, Heteroaryl, Aralkyl, wie $C_7$- bis $C_{20}$-Aralkyl, und Alkylaryl, wie $C_7$- bis $C_{20}$-Alkylaryl, jeweils wie oben definiert,

X:

- $CH_2$, $CHR^5$, Sauerstoff (O), Schwefel (S) oder $NR^5$, bevorzugt $CH_2$ und O,
  Y:

- $N(R^{10})_2$, bevorzugt $NH_2$ und $N(CH_3)_2$,

- Hydroxy (OH),

- $C_{2-20}$-Alkylaminoalkyl, bevorzugt $C_{2-16}$-Alkylaminoalkyl, wie Methylaminomethyl, 2-Methylaminoethyl, Ethylaminomethyl, 2-Ethylaminoethyl und 2-(iso-Propylamino)ethyl,

- $C_{3-20}$-Dialkylaminoalkyl, bevorzugt $C_{3-16}$-Dialkylaminoalkyl, wie Dimethylaminomethyl, 2-Dimethylaminoethyl, 2-Diethylaminoethyl, 2-(Di-n-propylamino)ethyl und 2-(Di-iso-propylamino)ethyl,

Z:

- $CH_2$, $CHR^5$, O, $NR^5$ oder $NCH_2CH_2OH$,
  j, l:
- eine ganze Zahl von 1 bis 4 (1, 2, 3 oder 4), bevorzugt 2 und 3, besonders bevorzugt 2,
  k, m, q:
- eine ganze Zahl von 1 bis 4 (1, 2, 3 oder 4), bevorzugt 2, 3 und 4, besonders bevorzugt 2 und 3,
  n:
- eine ganze Zahl von 1 bis 30, bevorzugt eine ganze Zahl von 1 bis 8 (1, 2, 3, 4, 5, 6, 7 oder 8), besonders bevorzugt eine ganze Zahl von 1 bis 6.

Als Alkohole eignen sich unter den o.g. Voraussetzungen praktisch alle primären und sekundären Alkohole mit aliphatischer OH-Funktion. Die Alkohole können geradkettig, verzweigt oder zyklisch sein. Sekundäre Alkohole

werden ebenso aminiert wie primäre Alkohole. Die Alkohole können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Alkohole, wie z. B. Diole oder Triole, besonders Glykole, aminiert werden, so hat man es über die Steuerung der Reaktionsbedingungen in der Hand, bevorzugt Aminoalkohole, zyklische Amine oder mehrfach aminierte Produkte zu erhalten.

Die Aminierung von 1,2-Diolen führt je nach Wahl der Reaktionsbedingungen besonders zu 1-Amino-2-hydroxy- oder 1,2-Diamino-Verbindungen.

Die Aminierung von 1,4-Diolen führt je nach Wahl der Reaktionsbedingungen zu 1-Amino-4-hydroxy-, 1,4-Diamino-Verbindungen oder zu fünfgliedrigen Ringen mit einem Stickstoffatom (Pyrrolidine).

Die Aminierung von 1,6-Diolen führt je nach Wahl der Reaktionsbedingungen zu 1-Amino-6-hydroxy-, 1,6-Diamino-Verbindungen oder zu siebengliedrigen Ringen mit einem Stickstoffatom (Hexamethylenimine).

Die Aminierung von 1,5-Diolen führt je nach Wahl der Reaktionsbedingungen zu 1-Amino-5-hydroxy-, 1,5-Diamino-Verbindungen oder zu sechsgliedrigen Ringen mit einem Stickstoffatom (Piperidine, 1,5-Di-piperidinyl-pentane).

Aus Diglykol (DEG) kann demnach durch Aminierung mit $NH_3$ Monoaminodiglykol (= ADG = $H_2N\text{-}CH_2CH_2\text{-}O\text{-}CH_2CH_2\text{-}OH$), Diaminodiglykol ($H_2N\text{-}CH_2CH_2\text{-}O\text{-}CH_2CH_2\text{-}NH_2$) oder Morpholin erhalten werden. Besonders bevorzugt ist hier das ADG als Verfahrensprodukt.

Aus Diethanolamin wird entsprechend besonders bevorzugt Piperazin erhalten. Aus Triethanolamin kann N-(2-Hydroxyethyl)-piperazin erhalten werden.

Bevorzugt werden beispielsweise die folgenden Alkohole aminiert:

Methanol, Ethanol, n-Propanol, Isopropanol, n-Butanol, iso-Butanol, n-Pentanol, n-Hexanol, 2-Ethylhexanol, Tridecanol, Stearylalkohol, Palmitylalkohol, Cyclobutanol, Cyclopentanol, Cyclohexanol, Benzylalkohol, 2-Phenyl-ethanol, 2-(p-Methoxyphenyl)-ethanol, 2-(3,4-Dimethoxyphenyl)ethanol, 1-Phenyl-3-butanol, Ethanolamin, n-Propanolamin, Isopropanolamin, 2-Amino-1-propanol, 1-Methoxy-2-propanol, 3-Amino-2,2-dimethyl-1-propanol, n-Pentanolamin (1-Amino-5-pentanol), n-Hexanolamin (1-Amino-6-hexanol), Ethanolamin, Diethanolamin, Triethanolamin, N-Alkyldiethanolamine, Diisopropanolamin, 3-(2-Hydroxyethylamino)propan-1-ol, 2-(N,N-Dimethylamino)ethanol, 2-(N,N-Diethylamino)ethanol, 2-(N,N-Di-n-propylamino)-ethanol, 2-(N,N-Di-iso-propylamino)ethanol, 2-(N,N-Di-n-butylamino)ethanol, 2-(N,N-Di-iso-butylamino)ethanol, 2-(N,N-Di-sec.-butylamino)ethanol, 2-(N,N-Di-tert.-butylamino)ethanol, 3-(N,N-Dimethylamino)propanol, 3-(N,N-Diethylamino)propanol, 3-(N,N-Di-n-propylamino)propanof, 3-(N,N-Di-iso-propylamino)propanol, 3-(N,N-Di-n-butylamino)propanol, 3-(N,N-Di-iso-butylamino)propanol, 3-(N,N-Di-sec.-butylamino)-propanol, 3-(N,N-Di-tert.-butylamino)propanol, 1-Dimethylamino-pentanol-4, 1-Diethylamino-pentanol-4, Ethylenglykol, 1,2-Propylenglykol, 1,3-Propylenglykol, Diglykol, 1,4-Butandiol, 1,5-Pentandiol, 1,6-Hexandiol, 2,2-Bis[4-hydroxycyclohexyl]propan, Methoxyethanol, Propoxyethanol, Butoxyethanol, Polypropylalkohole, Polyethylenglykolether, Polypropylenglykolether und Polybutylenglykolether. Die letztgenannten Polyalkylenglykolether werden bei der erfindungsgemäßen Umsetzung durch Umwandlung ihrer freien Hydroxylgruppen zu den entsprechenden Aminen umgewandelt.

Besonders bevorzugte Alkohole sind Methanol, Ethanol, n-Propanol, i-Propanol, n-Butanol, sek.-Butanol, 1,4-Butandiol, 1,5-Pentandiol, 1,6-Hexandiol, 2-Ethylhexanol, Cyclohexanol, Fettalkohole, Ethylenglykol, Diethylenglykol (DEG), Triethylenglykol (TEG), 2-(2-Dimethylamino-ethoxy)ethanol, N-Methyldiethanolamin und 2-(2-Dimethylaminoethoxy)ethanol.

Als im erfindungsgemäßen Verfahren einsetzbare Ketone eignen sich unter den o.g. Voraussetzungen praktisch alle aliphatischen und aromatischen Ketone. Die aliphatischen Ketone können geradkettig, verzweigt oder zyklisch sein, die Ketone können Heteroatome enthalten. Die Ketone können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Ketone aminiert werden, so hat man es über die Steuerung der Reaktionsbedingungen in der Hand, Aminoketone, Aminoalkohole, cyclische Amine oder mehrfach aminierte Produkte zu erhalten.

Bevorzugt werden beispielsweise die folgenden Ketone aminierend hydriert:

Aceton, Ethylmethylketon, Methylvinylketon, Isobutylmethylketon, Butanon, 3-Methylbutan-2-on, Diethylketon, Tetralon, Acetophenon, p-Methyl-acetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

Als im erfindungsgemäßen Verfahren einsetzbare Aldehyde eignen sich unter den o.g. Voraussetzungen praktisch alle aliphatischen und aromatischen Aldehyde. Die aliphatischen Aldehyde können geradkettig, verzweigt oder zyklisch sein, die Aldehyde können Heteroatome enthalten. Die Aldehyde können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Aldehyde aminierend hydriert:

Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl)acetaldehyd, (3,4-Dimethoxyphenyl)-acetaldehyd, 4-Formyltetrahydropyran, 3- Formyltetrahydrofuran, 5-Formylvaleronitril, Citronellal, Lysmeral, Acrolein, Methacrolein, Ethylacrolein, Citral, Crotonaldehyd, 3-Methoxypropionaldehyd, 3-Aminopropionaldehyd, Hydroxypivalinaldehyd, Dimethylolpropionaldehyd, Dimethylolbutyraldehyd, Furfural, Glyoxal, Glutaraldehyd sowie hydroformylierte Oligomere und Polymere, wie z. B. hydroformyliertes Polyisobuten (Polyisobutenaldehyd) oder durch Metathese von 1-Penten und Cyclopenten erhaltenes und hydroformyliertes Oligomer.

Als Aminierungsmittel bei der hydrierenden Aminierung von Alkoholen, Aldehyden oder Ketonen in Gegenwart von Wasserstoff können sowohl Ammoniak als auch primäre oder sekundäre, aliphatische oder cycloaliphatische oder aromatische Amine eingesetzt werden.

Bei Verwendung von Ammoniak als Aminierungsmittel wird die alkoholische Hydroxylgruppe bzw. die Aldehydgruppe bzw. die Ketogruppe zunächst in die primäre Aminogruppen (-$NH_2$) umgewandelt. Das so gebildete primäre Amin können mit weiterem Alkohol bzw. Aldehyd bzw. Keton zu dem entsprechenden sekundären Amin und diese wiederum mit weiterem Alkohol bzw. Aldehyd bzw. Keton zu dem entsprechenden, vorzugsweise symmetrischen, tertiären Amin reagieren. Je nach Zusammensetzung des Reaktionsansatzes oder des Eduktstroms (bei kontinuierlicher Fahrweise) und je nach den angewandten Reaktionsbedingungen - Druck, Temperatur, Reaktionszeit (Katalysatorbelastung) - lassen sich auf diese Weise je nach Wunsch bevorzugt primäre, sekundäre oder tertiäre Amine darstellen.

Aus mehrwertigen Alkoholen bzw. Di- oder Oligoaldehyden bzw. Di- oder Oligoketonen bzw. Ketoaldehyden lassen sich auf diese Weise durch intramolekulare hydrierende Aminierung zyklische Amine wie z.B. Pyrrolidine, Piperidine, Hexamethylenimine, Piperazine und Morpholine herstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet. Beispielsweise werden die folgenden Mono- und Dialkylamine als Aminierungsmittel verwendet: Monomethylamin, Dimethylamin, Monoethylamin, Diethylamin, n-Propylamin, Di-n-propyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Mit dem erfindungsgemäßen Verfahren besonders bevorzugt hergestellte Amine sind zum Beispiel Morpholin (aus Monoaminodiglykol), Monoaminodiglykol, Morpholin und/oder 2,2'-Dimorpholinodiethylether (DMDEE) (aus DEG und Ammoniak), 6-Dimethylaminohexanol-1 (aus Hexandiol und Dimethylamin (DMA)), Triethylamin (aus Ethanol und Diethylamin (DEA)), Dimethylethylamin (aus Ethanol und DMA), N-($C_{1-4}$-alkyl)morpholin (aus DEG und Mono($C_{1-4}$-alkyl) amin), N-($C_{1-4}$-alkyl)piperidin (aus 1,5-Pentandiol und Mono($C_{1-4}$-alkyl)amin), Piperazin und/oder Diethylentriamin (DETA) (aus N-(2-Aminoethyl)-ethanolamin (AEEA) und Ammoniak), N-Methylpiperazin (aus Diethanolamin und MMA), N, N'-Dimethylpiperazin (aus N-Methyldiethanolamin und MMA), 1,2-Ethylendiamin (EDA) und/oder Diethylentriamin (DETA) und/oder PIP (aus Monoethanolamin (MEOA) und Ammoniak), 2-Ethylhexylamin und Bis(2-Ethylhexyl)amin (aus 2-Ethylhexanol und $NH_3$), Tridecylamin und Bis(Tridecyl)amin (aus Tridecanol und $NH_3$), n-Octylamin (aus n-Octanol und $NH_3$), 1,2-Propylendiamin (aus 2-Hydroxy-propylamin und $NH_3$), 1-Diethylamino-4-aminopentan (aus 1-Diethylamino-4-hydroxypentan und $NH_3$), N,N-Di($C_{1-4}$-alkyl)cyclohexylamin (aus Cyclohexanon und/oder Cyclohexanol und Di ($C_{1-4}$-alkyl)amin), z.B. N,N-Dimethyl-N-cyclohexylamin (DMCHA), Polyisobutenamin (PIBA; mit z.B. n-1000) (aus Polyisobutenaldehyd und $NH_3$), N,N-Diisopropyl-N-ethylamin (Hünigbase) (aus N,N-Diisopropylamin und Acetaldehyd), N-Methyl-N-isopropylamin (MMIPA) (aus Monomethylamin und Aceton), n-Propylamine (wie Mono-/Di-n-propylamin, N, N-Dimethyl-N-n-propylamin (DMPA)) (aus Propionaldehyd und/oder n-Propanol und $NH_3$ bzw. DMA), N,N-Dimethyl-N-isopropylamin (DMIPA) (aus i-Propanol und/oderAceton und DMA), N,N-Dimethyl-N-butylamine (1-, 2- oder iso-Butanol und/oder Butanal, i-Butanal oder Butanon und DMA), 2-(2-Di($C_{1-4}$-alkyl)aminoethoxy)ethanol und/oder Bis(2-di($C_{1-4}$-alkyl)aminoethyl)ether (aus DEG und Di($C_{1-4}$-alkyl)amin), 1,2-Ethylendiamin (EDA), Monoethanolamin (MEOA), Diethylentriamin (DETA) und/oder Piperazin (PIP) (aus Monoethylenglykol (MEG) und Ammoniak), 1,8-Diamino-3,6-dioxa-octan und/oder 1-Amino-8-hydroxy-3,6-dioxa-octan (aus Triethylenglykol (TEG) und Ammoniak), 1-Methoxy-2-propylamin (1-Methoxy-isopropylamin, MOIPA) (aus 1-Methoxy-2-propanol und Ammoniak), N-Cyclododecyl-2,6-dimethylmorpholin (Dodemorph) (aus Cyclododecanon und/oder Cyclododecanol und 2,6-Dimethylmorpholin), Polyetheramin (aus entsprechendem Polyetheralkohol und Ammoniak). Die Polyetheralkohole sind z.B. Polyethylenglykole oder Polypropylenglykole mit einem Molekulargewicht im Bereich von 200 bis 5000 g/mol, die entsprechende Polyetheramine sind z.B. unter dem Handelsname PEA D230, D400, D2000, T403 oder T5000 von BASF erhältlich.

Beispiele

Beispiel 1

Herstellung von Aminierungskatalysator 1 (auf Basis von Ni-Co-Cu/$ZrO_2$ = Vergleichsversuch nach EP 963 975 A)

Eine wässrige Lösung aus Nickelnitrat, Kobaltnitrat, Kupfernitrat und Zirkoniumacetat, die 2,39 Gew.-% NiO, 2,39 Gew.-% CoO, 0,94 Gew.-% CuO und 2,82 Gew.-% $ZrO_2$ enthielt, wurde gleichzeitig in einem Rührgefäß in einem konstanten Strom mit einer 20 %igen wässrigen Natriumcarbonatlösung bei einer Temperatur von 70°C so gefällt, dass der mit einer Glaselektrode gemessene pH-Wert von 7,0 aufrechterhalten wurde. Die erhaltene Suspension wurde filtriert und der Filterkuchen mit voll entsalztem Wasser gewaschen bis die elektrische Leitfähigkeit des Filtrats ca. 20 μS betrug. Danach wurde der Filterkuchen bei einer Temperatur von 150°C in einem Trockenschrank oder einem Sprühtrockner getrocknet. Das auf diese Weise erhaltene Hydroxid-Carbonat-Gemisch wurde nun bei einer Temperatur von 450 bis 500 °C über einen Zeitraum von 4 Stunden getempert. Der so hergestellte Katalysator hatte die Zusammensetzung: 28 Gew.-% NiO, 28 Gew.-% CoO, 11 Gew.-% CuO und 33 Gew.-% $ZrO_2$. Der Katalysator wurde mit 3 Gew.-% Graphit vermischt und zu Tabletten verformt. Die oxidische Tabletten wurden reduziert. Die Reduktion wurde bei 280°C durchgeführt, wobei die Aufheizungsrate 3°C/Minute betrug. Zuerst wurde 50 Minuten mit 10 % $H_2$ in $N_2$ reduziert, anschließend 20 Minuten mit 25 % $H_2$ in $N_2$, dann 10 Minuten mit 50 % $H_2$ in $N_2$, dann 10 Minuten mit 75 % $H_2$ in $N_2$ und schließlich 3 Stunden mit 100 % $H_2$. Bei den %-Angaben handelt es sich jeweils um Volumen-%. Die Passivierung der reduzierten Katalysator wurde bei Raumtemperatur in verdünnter Luft (Luft in $N_2$ mit einem $O_2$-Gehalt von maximal 5 Vol.-%) durchgeführt.

Beispiel 2

Der Katalysator wurde analog Katalysator 1 hergestellt. Allerdings wurde die Menge des Nickel-Nitrats, Kupfer-Nitrats und Kobalt-Nitrats entsprechend verändert. Weiterhin wurde der Nitratlösung noch Eisennitrat zugefügt, so dass das nachfolgend angegebene Oxidgemisch erhalten wurde. Der so erhaltene Katalysator 2 hatte die Zusammensetzung wie in der Tabelle I dargestellt.

Beispiel 3

Der Katalysator wurde analog Katalysator 2 hergestellt. Allerdings wurde die Menge des Nickel-Nitrats, Kupfer-Nitrats, Eisen-Nitrats und Kobalt-Nitrats entsprechend verändert. Weiterhin wurde der Nitratlösung noch Bleinitrat zugefügt, so dass das nachfolgend angegebene Oxidgemisch erhalten wurde. Der so erhaltene Katalysator 3 hatte die Zusammensetzung wie in der Tabelle I dargestellt.

Beispiel 4

Der Katalysator wurde analog Katalysator 2 hergestellt. Allerdings wurde die Menge des Nickel-Nitrats, Kupfer-Nitrats, Eisen-Nitrats und Kobalt-Nitrats entsprechend verändert. Weiterhin wurde der Nitratlösung noch Zinndichlorid zugefügt, so dass das nachfolgend angegebene Oxidgemisch erhalten wurde. Der so erhaltene Katalysator 4 hatte die Zusammensetzung wie in der Tabelle I dargestellt.

Beispiel 5

Der Katalysator wird analog Katalysator 2 hergestellt. Allerdings wird die Menge des Nickel-Nitrats, Kupfer-Nitrats, Eisen-Nitrats und Kobalt-Nitrats entsprechend verändert. Weiterhin wird der 20-%igen Natriumcarbonatlösung zusätzlich Antimonoxid gelöst in konzentrierter wässriger KOH zugefügt, so dass das nachfolgend angegebene Oxidgemisch erhalten wurde. Der so erhaltene Katalysator 5 hatte die Zusammensetzung wie in der Tabelle I dargestellt.

Beispiel 6

Der Katalysator wird analog Katalysator 2 hergestellt. Allerdings wird die Menge des Nickel-Nitrats, Kupfer-Nitrats, Eisen-Nitrats und Kobalt-Nitrats entsprechend verändert. Weiterhin wird der Nitratlösung noch Bismuthnitrat zugefügt, so dass das nachfolgend angegebene Oxidgemisch erhalten wurde. Der so erhaltene Katalysator 6 hatte die Zusammensetzung wie in der Tabelle I dargestellt.

Beispiel 7

Aminierung von Diethylenglykol (DEG)

10 g des reduzierten Aminierungskatalysators in Form vom ca. 0,2 bis 1 mm Splitt wurde in einem 300 ml Autoklav zusammen mit 70 g Diethylenglykol (0,65 mol) vorgelegt. Dem Reaktionsgemisch wurde 34 g flüssiger Ammoniak (2 mol) zugeführt, der Autoklav wurde auf 50 bar mit Wasserstoff aufgepresst und auf 200°C aufgeheizt. Bei 200°C wurde noch mal mit Wasserstoff aufgepresst, wobei der Gesamtdruck auf 180 - 200 bar stieg. Der Autoklav wurde bei 200°C 12 Stunden unter Rühren gefahren. Zu verschiedenen Zeitpunkten wurden Proben vom Reaktionsgemisch gezogen und mittels GC-Chromatographie analysiert. Hierfür wurde eine 30 m lange GC Säule "RTX-5 Amine" verwendet, mit einem Temperaturprogramm: 80°C/15 Minuten, aufheizen auf 290°C in 30 Minuten, bei 290°C/15 Minuten.
Die Zusammensetzung der resultierenden Reaktionsgemische für die Katalysatoren der Beispiele 1 bis 6 ist der Tabelle I zu entnehmen.

Tabelle I:

| Katalysator* | | | | | | | Performance | | | | | | | | |
|---|---|---|---|---|---|---|---|---|---|---|---|---|---|---|---|
| # | Ni<br>% | Co<br>% | Cu<br>% | Fe<br>% | Dot. | Dot.<br>% | Zeit<br>Stunden | DEG Umsatz<br>GC % | Aminierungs-aktivität** mmol<br>DEG/gKat·h | Morpholin (MOR)<br>GC % | ADG<br>GC % | Verhältnis ADG/ Morpholin GC % / GC % | Gesamtselektivität ADG+MOR<br>GC % | MeOEt<br>GC % | MeOEt normiert<br>Σ GC % |
| 1 | 21,9 | 21,9 | 10,5 | -- | -- | -- | 2 | 56,2 | 1,83 | 18,2 | 26,3 | 1,45 | 79,2 | 0,20 | 0,35 |
| 2 | 29,3 | 3,4 | 10,0 | 2,2 | -- | -- | 2 | 51,3 | 1,66 | 12,6 | 27,2 | 2,16 | 77,6 | 0,22 | 0,43 |
| 3 | 32,5 | 4,1 | 9,8 | 1,0 | Pb | 1,4 | 4 | 48,02 | 0,94 | 8,8 | 32,0 | 3,62 | 85,1 | 0,08 | 0,17 |
| 4 | 31,0 | 3,6 | 10,0 | 0,7 | Sn | 2,8 | 2 | 47,3 | 1,54 | 15,0 | 23,9 | 1,59 | 82,2 | 0,05 | 0,11 |
| 5 | 31,0 | 3,7 | 9,9 | 1,1 | Sb | 0,8 | 4 | 49,6 | 1,18 | 9,8 | 30,1 | 3,07 | 80,4 | 0,08 | 0,17 |
| 6 | 34,0 | 4,1 | 10,3 | 1,1 | Bi | 0,7 | 2 | 63,8 | 2,08 | 18,4 | 31,5 | 1,71 | 78,2 | 0,2 | 0,31 |

* Katalysatorzusammensetzung in Gew.-%; Rest bis zu 100 Gew.-% ist ZrO2
** Aminierungsaktivität: DEG umgesetzt / gkat·h in 2-Std Probe DEG Diethylenglykol
MOR Morpholin
ADG Aminodiglykol
MeOEt Methoxyethanol

Aufarbeitung:

Die jeweiligen Reinprodukte können aus den wasserhaltigen Rohwaren durch Rektifikation bei Vakuum, Normaldruck oder erhöhtem Druck nach den bekannten Methoden erhalten werden. Die Reinprodukte fallen dabei entweder direkt in reiner Form an oder als Azeotrope mit Wasser. Wasserhaltige Azeotrope können durch eine Flüssig-flüssig-Extraktion mit konzentrierter Natronlauge vor oder nach der Reindestillation entwässert werden. Eine destillative Entwässerung in Gegenwart eines Schleppmittels nach bekannten Methoden ist auch möglich.

Für den Fall, dass die Rohware oder das aliphatische Amin in der Rohware kaum oder nicht mit Wasser mischbar sind, ist eine Entwässerung durch eine Trennung der organischen und der wässrigen Phase mit bekannten Methoden auch möglich.

Fazit:

Die Performance von Aminierungskatalysatoren konnte gegenüber dem Stand der Technik, unter Erhalt der guten Katalysatoraktivität, deutlich verbessert werden, indem man die chemische Zusammensetzung der Aktivmasse veränderte. Die Ausbeute an wirtschaftlich interessanten, linearen Aminierungsprodukten wie Aminodiglykol konnte erhöht und das Ausmaß der unerwünschten Decarbonylierung, das durch den Gehalt an Methoxyethanol bestimmt wird, verringert werden, dadurch, dass neben dem Gehalt an Cu, Ni, Co ein spezifischer Gehalt an Fe und ein spezifischer Gehalt an Sn, Pb, Sb bzw. Bi im Katalysator eingestellt wurde.

## Patentansprüche

1. Verfahren zur Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines zirkoniumdioxid-, kupfer- und nickelhaltigen Katalysators, **dadurch gekennzeichnet, dass** die katalytisch aktive Masse des Katalysators vor dessen Reduktion mit Wasserstoff sauerstoffhaltige Verbindungen des Zirkoniums, Kupfers, Nickels, im Bereich von 0,2 bis 40 Gew.-% sauerstoffhaltige Verbindungen des Kobalts, berechnet als CoO, im Bereich von 0,1 bis 5 Gew.-% sauerstoffhaltige Verbindungen des Eisens, berechnet als $Fe_2O_3$, und im Bereich von 0,1 bis 5 Gew.-% sauerstoffhaltige Verbindungen des Bleis, Zinns, Bismuths und/oder Antimons, jeweils berechnet als PbO, SnO, $Bi_2O_3$ bzw. $Sb_2O_3$, enthält.

2. Verfahren nach Anspruch 1, **dadurch gekennzeichnet, dass** die katalytisch aktive Masse des Katalysators vor dessen Reduktion mit Wasserstoff im Bereich von 0,3 bis 4,5 Gew.-% sauerstoffhaltige Verbindungen des Bleis, Zinns, Bismuths und/oder Antimons, jeweils berechnet als PbO, SnO, $Bi_2O_3$ bzw. $Sb_2O_3$, enthält.

3. Verfahren nach Anspruch 1, **dadurch gekennzeichnet, dass** die katalytisch aktive Masse des Katalysators vor dessen Reduktion mit Wasserstoff im Bereich von 0,5 bis 4 Gew.-% sauerstoffhaltige Verbindungen des Bleis, Zinns, Bismuths und/oder Antimons, jeweils berechnet als PbO, SnO, $Bi_2O_3$ bzw. $Sb_2O_3$, enthält.

4. Verfahren nach einem der vorhergehenden Ansprüche, **dadurch gekennzeichnet, dass** die katalytisch aktive Masse des Katalysators vor dessen Reduktion mit Wasserstoff im Bereich von 0,2 bis 4 Gew.-% sauerstoffhaltige Verbindungen des Eisens, berechnet als $Fe_2O_3$, enthält.

5. Verfahren nach einem der Ansprüche 1 bis 3, **dadurch gekennzeichnet, dass** die katalytisch aktive Masse des Katalysators vor dessen Reduktion mit Wasserstoff im Bereich von 0,5 bis 3 Gew.-% sauerstoffhaltige Verbindungen des Eisens, berechnet als $Fe_2O_3$, enthält.

6. Verfahren nach einem der vorhergehenden Ansprüche, **dadurch gekennzeichnet, dass** die katalytisch aktive Masse des Katalysators vor dessen Reduktion mit Wasserstoff im Bereich von 1 bis 25 Gew.-% sauerstoffhaltige Verbindungen des Kobalts, berechnet als CoO, enthält.

7. Verfahren nach einem der Ansprüche 1 bis 5, **dadurch gekennzeichnet, dass** die katalytisch aktive Masse des

Katalysators vor dessen Reduktion mit Wasserstoff
im Bereich von 2 bis 10 Gew.-% sauerstoffhaltige Verbindungen des Kobalts, berechnet als CoO, enthält.

8. Verfahren nach einem der vorhergehenden Ansprüche, **dadurch gekennzeichnet, dass** die katalytisch aktive Masse des Katalysators vor dessen Reduktion mit Wasserstoff im Bereich von

   20 bis 85 Gew.-% sauerstoffhaltige Verbindungen des Zirkoniums, berechnet als $ZrO_2$,
   0,2 bis 25 Gew.-% sauerstoffhaltige Verbindungen des Kupfers, berechnet als CuO, und
   0,2 bis 45 Gew.-% sauerstoffhaltige Verbindungen des Nickels, berechnet als NIO, enthält.

9. Verfahren nach einem der Ansprüche 1 bis 7, **dadurch gekennzeichnet, dass** die katalytisch aktive Masse des Katalysators vor dessen Reduktion mit Wasserstoff im Bereich von

   25 bis 70 Gew.-% sauerstoffhaltige Verbindungen des Zirkoniums, berechnet als $ZrO_2$,
   3 bis 20 Gew.-% sauerstoffhaltige Verbindungen des Kupfers, berechnet als CuO, und
   10 bis 40 Gew.-% sauerstoffhaltige Verbindungen des Nickels, berechnet als NiO, enthält.

10. Verfahren nach einem der Ansprüche 1 bis 7, **dadurch gekennzeichnet, dass** die katalytisch aktive Masse des Katalysators vor dessen Reduktion mit Wasserstoff im Bereich von

   30 bis 60 Gew.-% sauerstoffhaltige Verbindungen des Zirkoniums, berechnet als $ZrO_2$,
   5 bis 15 Gew.-% sauerstoffhaltige Verbindungen des Kupfers, berechnet als CuO, und
   25 bis 35 Gew.-% sauerstoffhaltige Verbindungen des Nickels, berechnet als NiO, enthält.

11. Verfahren nach einem der vorhergehenden Ansprüche, **dadurch gekennzeichnet, dass** die katalytisch aktive Masse des Katalysators vor dessen Reduktion mit . Wasserstoff im Bereich von

   2 bis 10 Gew.-% sauerstoffhaltige Verbindungen des Kobalts, berechnet als CoO, und
   0,1 bis 5,0 Gew.%, bevorzugt 0,3 bis 4,5 Gew.%, besonders 0,5 bis 4 Gew.-%, sauerstoffhaltige Verbindungen des Bleis, berechnet als PbO, enthält.

12. Verfahren nach einem der vorhergehenden Ansprüche, **dadurch gekennzeichnet, dass** im Katalysator das Molverhältnis von Nickel zu Kupfer größer 1 ist.

13. Verfahren nach einem der vorhergehenden Ansprüche, **dadurch gekennzeichnet, dass** die katalytisch aktive Masse des Katalysators kein Rhenium und/oder Ruthenium enthält.

14. Verfahren nach einem der vorhergehenden Ansprüche, **dadurch gekennzeichnet, dass** die katalytisch aktive Masse des Katalysators kein Zink enthält.

15. Verfahren nach einem der vorhergehenden Ansprüche, **dadurch gekennzeichnet, dass** die katalytisch aktive Masse des Katalysators kein Silber und/oder kein Molybdän enthält.

16. Verfahren nach einem der Ansprüche 1 bis 12, **dadurch gekennzeichnet, dass** die katalytisch aktive Masse des Katalysators keine weitere katalytisch aktive Komponente, weder in elementarer noch in ionischer Form, enthält.

17. Verfahren nach einem der vorhergehenden Ansprüche, **dadurch gekennzeichnet, dass** man die Umsetzung bei einer Temperatur im Bereich von 80 bis 350 °C durchführt.

18. Verfahren nach einem der vorhergehenden Ansprüche, **dadurch gekennzeichnet, dass** man die Umsetzung in der Flüssigphase bei einem Absolutdruck im Bereich von 5 bis 30 MPa oder in der Gasphase bei einem Absolutdruck im Bereich von 0,1 bis 40 MPa durchführt.

19. Verfahren nach einem der vorhergehenden Ansprüche, **dadurch gekennzeichnet, dass** die Aminkomponente (Stickstoffverbindung) in der 0,90- bis 100-fachen molaren Menge bezogen auf den/das eingesetzte/n Alkohol, Aldehyd und/oder Keton eingesetzt wird.

20. Verfahren nach einem der Ansprüche 1 bis 18, **dadurch gekennzeichnet, dass** die Aminkomponente (Stickstoffverbindung) in der 1,0- bis 10-fachen molaren Menge bezogen auf den/das eingesetzte/n Alkohol, Aldehyd und/oder Keton eingesetzt wird.

**21.** Verfahren nach einem der vorhergehenden Ansprüche, **dadurch gekennzeichnet, dass** der Katalysator im Reaktor als Festbett angeordnet ist.

**22.** Verfahren nach einem der vorhergehenden Ansprüche, **dadurch gekennzeichnet, dass** es kontinuierlich durchgeführt wird.

**23.** Verfahren nach dem vorhergehenden Anspruch, **dadurch gekennzeichnet, dass** die Umsetzung in einem Rohrreaktor erfolgt.

**24.** Verfahren nach einem der beiden vorhergehenden Ansprüche, **dadurch gekennzeichnet, dass** die Umsetzung bei einer Kreisgasfahrweise erfolgt.

**25.** Verfahren nach.einem der vorhergehenden Ansprüche, **dadurch gekennzeichnet, dass** man den Alkohol, Aldehyd und/oder das Keton als wässrige Lösung einsetzt.

**26.** Verfahren nach einem der vorhergehenden Ansprüche, **dadurch gekennzeichnet, dass** man den Ammoniak, das primäre oder sekundäre Amin als wässrige Lösung einsetzt.

**27.** Verfahren nach einem der vorhergehenden Ansprüche zur Herstellung von Monoaminodiglykol (ADG) und Morpholin durch Umsetzung von Diethylenglykol (DEG) mit Ammoniak.

**28.** Verfahren nach einem der Ansprüche 1 bis 26 zur Herstellung von N-($C_{1-4}$-alkyl)-morpholin durch Umsetzung von Diethylenglykol (DEG) mit Mono($C_{1-4}$-alkyl)amin.

**29.** Verfahren nach einem der Ansprüche 1 bis 26 zur Herstellung von 2-(2-Di($C_{1-4}$-alkyl)aminoethoxy)ethahol und/oder Bis(2-di($C_{1-4}$-alkyl)aminoethyl)ether durch
Umsetzung von Diethylenglykol (DEG) mit Di($C_{1-4}$-alkyl)amin.

**30.** Verfahren nach einem der Ansprüche 1 bis 26 zur Herstellung von Monoethanolamin (MEOA) und/oder 1,2-Ethylendiamin (EDA) durch Umsetzung von Monoethylenglykol (MEG) mit Ammoniak.

**31.** Verfahren nach einem der Ansprüche 1 bis 26 zur Herstellung von 1,2-Ethylendiamin (EDA) durch Umsetzung von Monoethanolamin (MEOA) mit Ammoniak.

**32.** Verfahren nach einem der Ansprüche 1 bis 26 zur Herstellung eines Polyetheramins durch Umsetzung eines entsprechenden Polyetheralkohols mit Ammoniak.

**33.** Verfahren nach einem der Ansprüche 1 bis 26 zur Herstellung von Piperazin und/oder Diethylentriamin (DETA) durch Umsetzung von N-(2-Aminoethyl)-ethanolamin (AEEA) mit Ammoniak.

**34.** Verfahren nach einem der Ansprüche 1 bis 26 zur Herstellung von Polyiso-butenamin (PIBA) durch Umsetzung von Polyisobutenaldehyd mit Ammoniak.

**35.** Katalysator, **dadurch gekennzeichnet, dass** die katalytisch aktive Masse des Katalysators vor dessen Reduktion mit Wasserstoff sauerstoffhaltige Verbindungen des Zirkoniums, Kupfers, Nickels, im Bereich von 0,2 bis 40 Gew.-% sauerstoffhaltige Verbindungen des Kobalts, berechnet als CoO, im Bereich von 0,1 bis 5 Gew.-% sauerstoffhaltige Verbindungen des Eisens, berechnet als $Fe_2O_3$, und im Bereich von 0,1 bis 5 Gew.-% sauerstoffhaltige Verbindungen des Bleis, Zinns, Bismuths und/oder Antimons, jeweils berechnet als PbO, SnO, $Bi_2O_3$ bzw. $Sb_2O_3$, enthält.

**36.** Katalysator nach dem vorhergehenden Anspruch, gekennzeichnet wie in einem der Ansprüche 2 bis 16 definiert.

## Claims

**1.** A process for preparing an amine by reacting a primary or secondary alcohol, aldehyde and/or ketone with hydrogen

and a nitrogen compound selected from the group of ammonia, primary and secondary amines, in the presence of a zirconium dioxide-, copper- and nickel-containing catalyst, wherein the catalytically active composition of the catalyst, before its reduction with hydrogen, comprises oxygen compounds of zirconium, of copper, of nickel, in the range from 0.2 to 40% by weight of oxygen compounds of cobalt, calculated as CoO, in the range from 0.1 to 5% by weight of oxygen compounds of iron, calculated as $Fe_2O_3$, and in the range from 0.1 to 5% by weight of oxygen compounds of lead, of tin, of bismuth and/or of antimony, calculated in each case as PbO, SnO, $Bi_2O_3$ and $Sb_2O_3$ respectively.

**2.** The process according to claim 1, wherein the catalytically active composition of the catalyst, before its reduction with hydrogen, comprises in the range from 0.3 to 4.5% by weight of oxygen compounds of lead, of tin, of bismuth, and/or of antimony, calculated in each case as PbO, SnO, $Bi_2O_3$ and $Sb_2O_3$ respectively.

**3.** The process according to claim 1, wherein the catalytically active composition of the catalyst, before its reduction with hydrogen, comprises in the range from 0.5 to 4% by weight of oxygen compounds of lead, of tin, of bismuth and/or of antimony calculated in each case as PbO, SnO, $Bi_2O_3$ and $Sb_2O_3$ respectively.

**4.** The process according to any of the preceding claims, wherein the catalytically active composition of the catalyst, before its reduction with hydrogen, comprises in the range from 0.2 to 4% by weight of oxygen compounds of iron, calculated as $Fe_2O_3$.

**5.** The process according to any of claims 1 to 3, wherein the catalytically active composition of the catalyst, before its reduction with hydrogen, comprises in the range from 0.5 to 3% by weight of oxygen compounds of iron, calculated as $Fe_2O_3$.

**6.** The process according to any of the preceding claims, wherein the catalytically active composition of the catalyst, before its reduction with hydrogen, comprises in the range from 1 to 25% by weight of oxygen compounds of cobalt, calculated as CoO.

**7.** The process according to any of claims 1 to 5, wherein the catalytically active composition of the catalyst, before its reduction with hydrogen, comprises in the range from 2 to 10% by weight of oxygen compounds of cobalt, calculated as CoO.

**8.** The process according to any of the preceding claims, wherein the catalytically active composition of the catalyst, before its reduction with hydrogen, comprises in the range from 20 to 85% by weight of oxygen compounds of zirconium, calculated as $ZrO_2$, 0.2 to 25% by weight of oxygen compounds of copper, calculated as CuO, and 0.2 to 45% by weight of oxygen compounds of nickel, calculated as NiO.

**9.** The process according to any of claims 1 to 7, wherein the catalytically active composition of the catalyst, before its reduction with hydrogen, comprises in the range from

25 to 70% by weight of oxygen compounds of zirconium, calculated as $ZrO_2$,
3 to 20% by weight of oxygen compounds of copper, calculated as CuO, and
10 to 40% by weight of oxygen compounds of nickel, calculated as NiO.

**10.** The process according to any of claims 1 to 7, wherein the catalytically active composition of the catalyst, before its reduction with hydrogen, comprises in the range from

30 to 60% by weight of oxygen compounds of zirconium, calculated as $ZrO_2$,
5 to 15% by weight of oxygen compounds of copper, calculated as CuO, and
25 to 35% by weight of oxygen compounds of nickel, calculated as NiO.

**11.** The process according to any of the preceding claims, wherein the catalytically active composition of the catalyst, before its reduction with hydrogen, comprises in the range from 2 to 10% by weight of oxygen compounds of cobalt, calculated as CoO, and
0.1 to 5.0% by weight, preferably 0.3 to 4.5% by weight, particularly 0.5 to 4% by weight, of oxygen compounds of lead, calculated as PbO.

**12.** The process according to any of the preceding claims, wherein the molar ratio of nickel to copper in the catalyst is

greater than 1.

**13.** The process according to any of the preceding claims, wherein the catalytically active composition of the catalyst does not comprise any rhenium and/or ruthenium.

**14.** The process according to any of the preceding claims, wherein the catalytically active composition of the catalyst does not comprise any zinc.

**15.** The process according to any of the preceding claims, wherein the catalytically active composition of the catalyst does not comprise any silver and/or any molybdenum.

**16.** The process according to any of claims 1 to 12, wherein the catalytically active composition of the catalyst does not comprise any further catalytically active component, either in elemental or in ionic form.

**17.** The process according to any of the preceding claims, wherein the reaction is performed at a temperature in the range from 80 to 350°C.

**18.** The process according to any of the preceding claims, wherein the reaction is performed in the liquid phase at an absolute pressure in the range from 5 to 30 MPa or in the gas phase at an absolute pressure in the range from 0.1 to 40 MPa.

**19.** The process according to any of the preceding claims, wherein the amine component (nitrogen compound) is used in from 0.90 to 100 times the molar amount based on the alcohol, aldehyde and/or ketone used.

**20.** The process according to any of claims 1 to 18, wherein the amine component (nitrogen compound) is used in from 1.0 to 10 times the molar amount based on the alcohol, aldehyde and/or ketone used.

**21.** The process according to any of the preceding claims, wherein the catalyst is arranged in the reactor as a fixed bed.

**22.** The process according to any of the preceding claims, which is performed contiguously.

**23.** The process according to the preceding claim, wherein the reaction is effected in a tubular reactor.

**24.** The process according to either of the two preceding claims, wherein the reaction is effected in a cycle gas method.

**25.** The process according to any of the preceding claims, wherein the alcohol, aldehyde and/or the ketone is used as an aqueous solution.

**26.** The process according to any of the preceding claims, wherein the ammonia, the primary or secondary amine is used as an aqueous solution.

**27.** The process according to any of the preceding claims for preparing monoaminodiglycol (ADG) and morpholine by reacting diethylene glycol (DEG) with ammonia.

**28.** The process according to any of claims 1 to 26 for preparing N-($C_{1-4}$-alkyl) morpholine by reacting diethylene glycol (DEG) with mono($C_{1-4}$-alkyl)amine.

**29.** The process according to any of claims 1 to 26 for preparing 2-(2-di($C_{1-4}$-alkyl)aminoethoxy)ethanol and/or bis(2-di($C_{1-4}$-alkyl)aminoethyl) ether by reacting diethylene glycol (DEG) with di($C_{1-4}$-alkyl) amine.

**30.** The process according to any of claims 1 to 26 for preparing monoethanolamine (MEOA) and/or 1,2-ethylenediamine (EDA) by reacting monoethylene glycol (MEG) with ammonia.

**31.** The process according to any of claims 1 to 26 for preparing 1,2-ethylenediamine (EDA) by reacting monoethanolamine (MEOA) with ammonia.

**32.** The process according to any of claims 1 to 26 for preparing a polyetheramine by reacting a corresponding polyether alcohol with ammonia.

**33.** The process according to any of claims 1 to 26 for preparing piperazine and/or diethylenetriamine (DETA) by reacting N-(2-aminoethyl)ethanolamine (AEEA) with ammonia.

**34.** The process according to any of claims 1 to 26 for preparing polyisobutenamine (PIBA) by reacting polyisobutenaldehyde with ammonia and hydrogen.

**35.** A catalyst, wherein the catalytically active composition of the catalyst, before its reduction with hydrogen, comprises oxygen compounds of zirconium, of copper, of nickel, in the range from 0.2 to 40% by weight of oxygen compounds of cobalt, calculated as CoO, in the range from 0.1 to 5% by weight of oxygen compounds of iron, calculated as $Fe_2O_3$, and in the range from 0.1 to 5% by weight of oxygen compounds of lead, of tin, of bismuth and/or of antimony, calculated in each case as PbO, SnO, $Bi_2O_3$ and $Sb_2O_3$ respectively.

**36.** The catalyst according to the preceding claim, which is as defined in any of claims 2 to 16.

## Revendications

**1.** Procédé pour la préparation d'une amine par transformation d'un alcool primaire ou secondaire, d'un aldéhyde et/ou d'une cétone avec de l'hydrogène et un composé azoté, choisi dans le groupe formé par l'ammoniac, les amines primaires et secondaires, en présence d'un catalyseur contenant du dioxyde de zirconium, du cuivre et du nickel, **caractérisé en ce que** la masse catalytiquement active du catalyseur contient, avant sa réduction avec de l'hydrogène, des composés oxygénés du zirconium, du cuivre, du nickel, dans la plage de 0,2 à 40% en poids de composés oxygénés du cobalt, calculé sous forme de CoO, dans la plage de 0,1 à 5% en poids de composés oxygénés du fer, calculé sous forme de $Fe_2O_3$, et dans la plage de 0,1 à 5% en poids de composés oxygénés du plomb, de l'étain, du bismuth et/ou de l'antimoine, respectivement calculés sous forme de PbO, de SnO, de $Bi_2O_3$ ou, selon le cas, de $Sb_2O_3$.

**2.** Procédé selon la revendication 1, **caractérisé en ce que** la masse catalytiquement active du catalyseur contient, avant sa réduction avec de l'hydrogène, dans la plage de 0,3 à 4,5% en poids de composés oxygénés du plomb, de l'étain, du bismuth et/ou de l'antimoine, respectivement calculés sous forme de PbO, de SnO, de $Bi_2O_3$ ou, selon le cas, de $Sb_2O_3$.

**3.** Procédé selon la revendication 1, **caractérisé en ce que** la masse catalytiquement active du catalyseur contient, avant sa réduction avec de l'hydrogène, dans la plage de 0,5 à 4% en poids de composés oxygénés du plomb, de l'étain, du bismuth et/ou de l'antimoine, respectivement calculés sous forme de PbO, de SnO, de $Bi_2O_3$ ou, selon le cas, de $Sb_2O_3$.

**4.** Procédé selon l'une quelconque des revendications précédentes, **caractérisé en ce que** la masse catalytiquement active du catalyseur contient, avant sa réduction avec de l'hydrogène, dans la plage de 0,2 à 4% en poids de composés oxygénés du fer, calculé sous forme de $Fe_2O_3$.

**5.** Procédé selon l'une quelconque des revendications 1 à 3, **caractérisé en ce que** la masse catalytiquement active du catalyseur contient, avant sa réduction avec de l'hydrogène, dans la plage de 0,5 à 3% en poids de composés oxygénés du fer, calculé sous forme de $Fe_2O_3$.

**6.** Procédé selon l'une quelconque des revendications précédentes, **caractérisé en ce que** la masse catalytiquement active du catalyseur contient, avant sa réduction avec de l'hydrogène, dans la plage de 1 à 25% en poids de composés oxygénés du cobalt, calculé sous forme de CoO.

**7.** Procédé selon l'une quelconque des revendications 1 à 5, **caractérisé en ce que** la masse catalytiquement active du catalyseur contient, avant sa réduction avec de l'hydrogène, dans la plage de 2 à 10% en poids de composés oxygénés du cobalt, calculé sous forme de CoO.

**8.** Procédé selon l'une quelconque des revendications précédentes, **caractérisé en ce que** la masse catalytiquement active du catalyseur contient, avant sa réduction avec l'hydrogène, dans la plage de 20 à 85 % en poids de composés oxygénés du zirconium, calculé sous forme de $ZrO_2$,

0,2 à 25 % en poids de composés oxygénés du cuivre, calculé sous forme de CuO, et

0,2 à 45 % en poids de composés oxygénés du nickel, calculé sous forme de NiO.

**9.** Procédé selon l'une quelconque des revendications 1 à 7, **caractérisé en ce que** la masse catalytiquement active du catalyseur contient, avant sa réduction avec l'hydrogène, dans la plage de 25 à 70 % en poids de composés oxygénés du zirconium, calculé sous forme de $ZrO_2$,

3 à 20 % en poids de composés oxygénés du cuivre, calculé sous forme de CuO, et
10 à 40 % en poids de composés oxygénés du nickel, calculé sous forme de NiO.

**10.** Procédé selon l'une quelconque des revendications 1 à 7, **caractérisé en ce que** la masse catalytiquement active du catalyseur contient, avant sa réduction avec l'hydrogène, dans la plage de 30 à 60 % en poids de composés oxygénés du zirconium, calculé sous forme de $ZrO_2$,

5 à 15 % en poids de composés oxygénés du cuivre, calculé sous forme de CuO, et
25 à 35 % en poids de composés oxygénés du nickel, calculé sous forme de NiO.

**11.** Procédé selon l'une quelconque des revendications précédentes, **caractérisé en ce que** la masse catalytiquement active du catalyseur contient, avant sa réduction avec l'hydrogène, dans la plage de 2 à 10 % en poids de composés oxygénés du cobalt, calculé sous forme de CoO, et

0,1 à 5,0% en poids, de préférence 0,3 à 4,5% en poids, de manière particulièrement préférée 0,5 à 4% en poids, de composés oxygénés du plomb, calculé sous forme de PbO.

**12.** Procédé selon l'une quelconque des revendications précédentes, **caractérisé en ce que** dans le catalyseur, le rapport molaire de nickel à cuivre est supérieur à 1.

**13.** Procédé selon l'une quelconque des revendications précédentes, **caractérisé en ce que** la masse catalytiquement active du catalyseur ne contient pas de rhénium et/ou de ruthénium.

**14.** Procédé selon l'une quelconque des revendications précédentes, **caractérisé en ce que** la masse catalytiquement active du catalyseur ne contient pas de zinc.

**15.** Procédé selon l'une quelconque des revendications précédentes, **caractérisé en ce que** la masse catalytiquement active du catalyseur ne contient pas d'argent et/ou pas de molybdène.

**16.** Procédé selon l'une quelconque des revendications 1 à 12, **caractérisé en ce que** la masse catalytiquement active du catalyseur ne contient pas d'autres composants catalytiquement actifs, ni sous forme élémentaire, ni sous forme ionique.

**17.** Procédé selon l'une quelconque des revendications précédentes, **caractérisé en ce qu'**on réalise la transformation à une température dans la plage de 80 à 350°C.

**18.** Procédé selon l'une quelconque des revendications précédentes, **caractérisé en ce qu'**on réalise la transformation en phase liquide à une pression absolue dans la plage de 5 à 30 MPa ou en phase gazeuse à une pression absolue dans la plage de 0,1 à 40 MPa.

**19.** Procédé selon l'une quelconque des revendications précédentes, **caractérisé en ce que** le composant amine (composé azoté) est utilisé en une quantité représentant 0,90 à 100 fois la quantité molaire par rapport à l'alcool/aux alcools, à l'aldéhyde et/ou à la cétone utilisé(e)(s).

**20.** Procédé selon l'une quelconque des revendications 1 à 18, **caractérisé en ce que** le composant amine (composé azoté) est utilisé en une quantité représentant 1,0 à 10 fois la quantité molaire par rapport à l'alcool/aux alcools cycloaliphatique(s), à l'aldéhyde et/ou à la cétone utilisé(e)(s).

**21.** Procédé selon l'une quelconque des revendications précédentes, **caractérisé en ce que** le catalyseur est disposé dans le réacteur sous forme de lit fixe.

**22.** Procédé selon l'une quelconque des revendications précédentes, **caractérisé en ce qu'**il est réalisé en continu.

23. Procédé selon la revendication précédente, **caractérisé en ce que** la transformation a lieu dans un réacteur tubulaire.

24. Procédé selon l'une quelconque des deux revendications précédentes, **caractérisé en ce que** la transformation a lieu dans un mode opératoire avec un gaz en circulation.

25. Procédé selon l'une quelconque des revendications précédentes, **caractérisé en ce qu'**on utilise l'alcool, l'aldéhyde et/ou la cétone sous forme de solution aqueuse.

26. Procédé selon l'une quelconque des revendications précédentes, **caractérisé en ce qu'**on utilise l'ammoniac, l'amine primaire ou secondaire sous forme de solution aqueuse.

27. Procédé selon l'une quelconque des revendications précédentes pour la préparation de monoaminodiglycol (ADG) et de morpholine par transformation de diéthylèneglycol (DEG) avec de l'ammoniac.

28. Procédé selon l'une quelconque des revendications 1 à 26 pour la préparation de N-($C_{1-4}$-alkyl)-morpholine par transformation de diéthylèneglycol (DEG) avec de la mono($C_{1-4}$-alkyl)amine.

29. Procédé selon l'une quelconque des revendications 1 à 26 pour la préparation de 2-(2-di ($C_{1-4}$-alkyl) aminoéthoxy) éthanol et/ou de bis (2-di($C_{1-4}$-alkyl)aminoéthyléther par transformation de diéthylèneglycol (DEG) avec de la di ($C_{1-4}$-alkyl)amine.

30. Procédé selon l'une quelconque des revendications 1 à 26 pour la préparation de monoéthanolamine (MEOA) et/ou de 1,2-éthylènediamine (EDA) par transformation de monoéthylèneglycol (MEG) avec de l'ammoniac.

31. Procédé selon l'une quelconque des revendications 1 à 26 pour la préparation de 1,2-éthylènediamine (EDA) par transformation de monoéthanolamine (MEOA) avec de l'ammoniac.

32. Procédé selon l'une quelconque des revendications 1 à 26 pour la préparation d'une polyétheramine par transformation d'un polyétheralcool correspondant avec de l'ammoniac.

33. Procédé selon l'une quelconque des revendications 1 à 26 pour la préparation de pipérazine et/ou de diéthylènetriamine (DETA) par transformation de N-(2-aminoéthyl)éthanolamine (AEEA) avec de l'ammoniac.

34. Procédé selon l'une quelconque des revendications 1 à 26 pour la préparation de polyisobutène-amine (PIBA) par transformation de polyisobutène-aldéhyde avec de l'ammoniac.

35. Catalyseur, **caractérisé en ce que** la masse catalytiquement active du catalyseur contient, avant sa réduction avec de l'hydrogène, des composés oxygénés du zirconium, du cuivre, du nickel, dans la plage de 0,2 à 40% en poids de composés oxygénés du cobalt, calculé sous forme de CoO, dans la plage de 0,1 à 5% en poids de composés oxygénés du fer, calculé sous forme de $Fe_2O_3$, et dans la plage de 0,1 à 5% en poids de composés oxygénés du plomb, de l'étain, du bismuth et/ou de l'antimoine, respectivement calculés sous forme de PbO, de SnO, de $Bi_2O_3$ ou, selon le cas, de $Sb_2O_3$.

36. Catalyseur selon la revendication précédente, **caractérisé en ce qu'**il est défini comme dans l'une quelconque des revendications 2 à 16.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3275554 A
- DE 2125039 A
- DE 3611230 A
- WO 06069673 A1
- US 4153581 A, Habermann
- US 4152353 A, Dow
- EP 382049 A1
- EP 963975 A1
- EP 1106600 A2
- WO 031076386 A
- EP 1431271 A1
- WO 03051508 A1
- WO 2007036496 A
- EP 06117249 A
- EP 06117251 A
- EP 06117253 A
- EP 06117259 A
- EP 06117243 A
- WO 2008006750 A
- WO 2008006748 A
- WO 2008006752 A
- WO 2008006749 A
- WO 2008006754 A
- WO 06117249 A
- WO 06117251 A
- WO 06117253 A
- EP 1312600 A
- EP 1312599 A
- EP 963975 A


### In der Beschreibung aufgeführte Nicht-Patentliteratur


- Fixed-Bed Reactors. Ullmann's Encyclopedia of Industrial Chemistry. vol. 4, 199-238
- **A. B. Stiles.** Catalyst Manufacture - Laboratory and Commercial Preparations. Marcel Dekker, 1983